# EUROPÄISCHE PATENTSCHRIFT

(11) **EP 1 595 887 B1**
(45) Veröffentlichungstag und Bekanntmachung des Hinweises auf die Patenterteilung: **25.10.2006**
(21) Anmeldenummer: 05009328.5
(22) Anmeldetag: 28.04.2005
(51) Int. Cl.: C07F 9/655, B01J 31/24

(54) **Chirale C2-symmetrische Biphenyle, deren Herstellung sowie Metallkomplexe enthaltend diese Liganden und deren Verwendung als Katalysatoren in chirogenen Synthesen**
Chiral C2-symmetric biphenyls, preparation thereof as well as metal complexes containing these ligands and the use as catalysts in chirogene synthesis
Biphényls chiraux C2-symmetriques, leur préparation ainsi que les complexes métalliques contentant ces ligands et leur usage comme catalysateurs pour la synthése chirogéne

(30) Priorität: 06.05.2004 DE 102004022397
(43) Veröffentlichungstag der Anmeldung: 16.11.2005
(73) Patentinhaber: Consortium für elektrochemische Industrie GmbH, 81379 München (DE)
(72) Erfinder: Peschko, Christian, Dr., 81543 München (DE); Popp, Alfred, Dr., 82008 Unterhaching (DE); Stohrer, Jürgen, Dr., 82049 Pullach (DE)
(74) Vertreter: Fränkel, Robert

(56) Entgegenhaltungen:
- EP-A- 0 926 152
- EP-A- 1 176 135
- WO-A-03/029259
- QUI, L. ET AL.: "Synthesis of Novel Diastereomeric Diphosphine Ligands and Their Applications in Asymmetric Hydrogenation Reactions" ORGANIC LETTERS, Bd. 4, Nr. 26, 2002, Seiten 4599-4602, XP002332185 & DATABASE BEILSTEIN [Online] BEILSTEIN INSTITUTE FOR ORGANIC CHEMISTRY, FRANKFURT-MAIN, DE; 25. Juli 2003 (2003-07-25), gefunden im XFIRE Database accession no. BRN 9347836; BRN 9353916; BRN 9326073
- SALIMBENI A ET AL: "ON THE REACTION OF ALPHA-(2-HYDROXYPHENOXY)ALKYLKETONES WITH DIMETHYLSULPHOXONIUM METHYLIDE A NOVEL ROUTE TO 2-SUBSTITUTED-2,3-DIHYDRO-2-HYDROXYMETHYL- 1,4-BENZODIOXINS" JOURNAL OF HETEROCYCLIC CHEMISTRY, HETERO CORP., TAMPA, FL, US, Bd. 25, Nr. 3, Mai 1988 (1988-05), Seiten 943-947, XP002946850 ISSN: 0022-152X
- DATABASE BEILSTEIN [Online] BEILSTEIN INSTITUTE FOR ORGANIC CHEMISTRY, FRANKFURT-MAIN, DE; 1992, XP002332186 Database accession no. BRN 4039504; BRN 1382653; BRN 1574581; BRN 1372890 & ROONEY, C.S.ET AL.: CAN. J. CHEM., Bd. 53, 1975, Seiten 2279-2292,
- UCHIRO H ET AL: "Asymmetric total synthesis of 9-methoxystrobilurin K" TETRAHEDRON LETTERS, ELSEVIER SCIENCE PUBLISHERS, AMSTERDAM, NL, Bd. 42, Nr. 27, 2. Juli 2001 (2001-07-02), Seiten 4531-4534, XP004245736 ISSN: 0040-4039
- DATABASE BEILSTEIN [Online] BEILSTEIN INSTITUTE FOR ORGANIC CHEMISTRY, FRANKFURT-MAIN, DE; 16. Mai 2000 (2000-05-16), XP002332187 Database accession no. BRN 8406257 & MATTSON, R.J. ET AL.: J. ORG. CHEM., Bd. 64, Nr. 21, 1999, Seiten 8004-8007,
- DATABASE BEILSTEIN [Online] BEILSTEIN INSTITUTE FOR ORGANIC CHEMISTRY, FRANKFURT-MAIN, DE; 29. November 1988 (1988-11-29), XP002332188 Database accession no. BRN 124256 & SVENSON, L.-A.: ACTA CHEM. SCAND., Bd. 26, 1972, Seiten 2372-2384,
- MAMOUNI R ET AL: "Synthesis of 2H-1,5-benzodioxepin and 2,5-dihydro-1,6-benzodioxocin derivatives via ring-closing metathesis reaction" TETRAHEDRON LETTERS, ELSEVIER SCIENCE PUBLISHERS, AMSTERDAM, NL, Bd. 45, Nr. 12, 15. März 2004 (2004-03-15), Seiten 2631-2633, XP004492797 ISSN: 0040-4039

## Beschreibung

Die Erfindung betrifft die Bereitstellung einer neuen Klasse C₂-symmetrischer Biaryldiphosphine, deren Verwendung als Liganden zur Herstellung von Metallkomplexen, Metallkomplexe enthaltend diese Liganden, die Verwendung dieser Metallkomplexe als Katalysatoren in der organischen Synthese, sowie katalytische Verfahren unter Verwendung dieser Metallkomplexe.

Die vorliegende Erfindung betrifft insbesondere neue racemische, enantiomerenreine oder enantiomerenangereicherte Biaryldiphosphine (1,1'-Bis-2,2'-phosphine), die als zweizähnige Liganden bei der Herstellung von Metallkomplexen Verwendung finden, und die Verwendung dieser Metallkomplexe als Katalysatoren in asymmetrischen Reaktionen (chirogenen Synthesen).

Enantiomerenreine Derivate dienen als Ausgangsmaterialien bzw. Zwischenprodukte bei der Synthese von Agrochemikalien und Pharmazeutika. Viele dieser Verbindungen werden zur Zeit als Racemat oder Diastereomerengemisch hergestellt und vermarktet. In vielen Fällen wird der gewünschte physiologische Effekt aber nur von einem Enantiomer oder Diastereomer bewirkt. Das andere Isomer ist im günstigsten Fall inaktiv, es kann aber auch dem gewünschten Effekt entgegenwirken oder sogar toxisch sein. Verfahren zur Trennung von Racematen oder Diastereomerengemischen werden deshalb immer wichtiger für die Darstellung hochenantiomerenreiner Verbindungen.

Alternativ kann ein stereogenes Zentrum in Molekülen gezielt aufgebaut werden - man spricht in diesem Zusammenhang von stereoselektiven Synthesen, wobei das Prinzip solcher Reaktionen auf der Tatsache beruht, dass die zwei möglichen Enantiomere eines chiralen Produktes in ungleichen Mengen gebildet werden.

In sog. enantioselektiven oder asymmetrischen Synthesen werden unter Zuhilfenahme chiraler Katalysatoren und der dadurch erreichten optischen Induktion optisch reine bzw. enantiomerenangereicherte Produkte direkt bei der Darstellung erhalten, ohne dass eine anschließende Racematspaltung notwendig wird.

Eine Reihe der aus dem Stand der Technik eingesetzten chiralen Katalysatoren besteht aus einem metallischen Zentrum, an das chirale Liganden koordiniert sind.

Insbesondere spielt die sog. axiale Chiralität eine große Rolle, die bei Molekülen der Punktgruppen *C*_{*n*} und *D*_{*n*} auftritt, wobei wiederum die dissymmetrischen Binaphthyl- oder Biphenyl-Liganden und deren Metall-Komplexe besonders häufig eingesetzt werden.

Binaphthyl- oder Biphenylsysteme bestehen aus zwei verknüpften Naphthalin- oder Phenyl-Einheiten. In der stereoselektiven Synthese finden 2,2'-substituierte 1,1'-Binaphthyle oder -phenyle als Liganden von Metallen breite Verwendung. Insbesondere das C₂-axialsymmetrische Binaphthyl-Gerüst ist dabei ein idealer Chiralitätsüberträger. Als koordinierende Substituenten in 2,2'-Position sind insbesondere Phosphingruppen zu nennen.

Ein chiraler Katalysator sollte insbesondere für eine großtechnische Anwendung idealerweise die folgenden Eigenschaften aufweisen:
■ Hohe Produktivität (S/C)
■ Hohe Aktivität (TOF)
■ Hohe Selektivität (ee)
■ Kostengünstige und unaufwändige Synthese des Katalysators

Für den industriellen Einsatz sollte das S/C-Verhältnis (molares Verhältnis Substrat/ Katalysator) im Bereich 1 000 - 50 000 und die Aktivität 500 h⁻¹ - 1 000 h⁻¹ liegen (Blaser, H.-U. und Studer, M., Chirality 11, 459-464 (1999)). Der Enantiomerenüberschuss (ee) sollte für pharmazeutische Verwendungen >98% ee sein.

Aus dem Stand der Technik sind eine Reihe von C₂-axialsymmetrischen Bisphosphinliganden, die für die Herstellung von Metallkomplexen verwendet werden, die wiederum als Katalysatoren bei der (asymmetrischen) Hydrierung, Carbonylierung, Hydrosilylierung oder C-C-Bindungsknüpfung verwendet werden, bekannt.

Besonders substituierte C₂-axialsymmetrische Biarylderivate, die an ein Übergangsmetall wie z. B. Ruthenium, Rhodium, Iridium oder Palladium koordiniert sind, eignen sich als Katalysatoren bei asymmetrischen Reaktionen. Erwähnt seien hier z. B. 2,2'-Bisdiphenylphosphino-[1,1']binaphthyl **(BINAP)** [EP 174057 B1, EP 245959 B1, EP 295109 B1, EP 295890 B1,EP 339764 B1], 2,2'-Bis(diphenylphosphin)-3,3'-dibenzo[b]thiophen **(BITIANP)** [EP 770085 B1], 5,5'-Bis-diphenylphosphino-[4,4']bi[benzo[1,3]dioxolyl] **(SEGPHOS)** [EP 850945 B1], 6,6'-Bis-diphenylphosphino-2,3,2',3'-tetrahydro-[5,5']bi[benzo[1,4]dioxinyl] **(SYNPHOS)** [WO 03/029259 Al] oder(Bis-4,4'-dibenzofuran-3,3'-diyl)-bis(diphenylphosphin) [EP 643065].

In der Vergangenheit wurden zahlreiche auf den grundlegenden BINAP-Arbeiten aufbauende Ligandensysteme zur Herstellung chiraler Metallkomplex-Katalysatoren entwickelt, die an spezielle Ansprüche bestimmter Reaktionen angepasst wurden. Insbesondere wurden auch Untersuchungen hinsichtlich der sterischen und elektronischen Einflüsse von Substituenten an BINAP-analogen Ligandensystemen vorgenommen. So ist im Stand der Technik der Einfluss anellierter Ringe mittlerer Größe auf Biarylliganden und der Einfluss zusätzlich eingeführter Stereozentren auf die chirale Induktion untersucht worden.

So wird beispielsweise die hohe Aktivität und Enantioselektivität der [5,5,6,6'-Bis(2*R*,4*R*-pentadioxy)]-(2,2-bis(diphenylphosphino)-(1,1')-biphenyl-Liganden der Gegenwart von vier asymmetrischen C-Atomen der 3,4-Dihydro-2H-1,5-dioxepin-Einheiten zugeschrieben [Qiu, L.; Qi, J.; Pai, C.-C.; Chan, S.; Zhou, Z.; Choi, M. C. K.; Chan, A. S. C.; Organic Letters 2002, Vol. 4, No. 26, 4599-4602]. Diese Systeme besitzen jedoch den Nachteil, dass bei ihrer Herstellung teure chirale Reagenzien eingesetzt werden müssen und sich als Produkte Diastereomerengemische bilden, die zunächst in einem zusätzlichen Schritt getrennt werden müssen, was weiter zu einer Verminderung der möglichen Ausbeute an reinen Isomeren führt.

Keiner der aus dem Stand der Technik bekannten Katalysatoren erfüllt bislang die oben genannten Kriterien, insbesondere hinsichtlich Aktivität, Selektivität und Zugänglichkeit für eine großtechnische Anwendung umfänglich.

Eine besondere Herausforderung an das Ligandensystem ist die Tatsache, dass insbesondere die Verdrillung entlang der C-C-Verknüpfung der Biaryleinheiten eine wesentliche Größe darstellt, die bezogen auf die Bedingungen des jeweils umzusetzenden Substrates ein individuelles Optimum besitzt.

Es bestand daher die Aufgabe, ein alternatives Ligandensystem bereitzustellen, das den Erfordernissen an ein großtechnisch einzusetzendes Katalysatorsystem gerecht wird und ferner einen breiten Anwendungsbereich hinsichtlich umzusetzender Substrate zulässt.

Die Aufgabe wurde gelöst durch die Bereitstellung einer neuen Klasse C₂-symmetrischer Biaryldiphosphine enthaltend ein synthetisch einfach zu variierendes, und somit an die individuellen Bedürfnisse des jeweils umzusetzenden Substrats anpassbares, anelliertes mindestens sieben Ringatome enthaltendes Ringsystem (Dioxacyclen), deren Verwendung als Liganden zur Herstellung von Metallkomplexen und die Verwendung dieser Komplexe als Katalysatoren in der chiralen Synthese.

Ein Gegenstand der Erfindung sind Verbindungen der allgemeinen Formel (I) 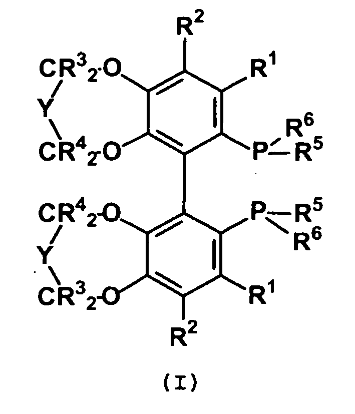 dadurch gekennzeichnet, dass
**R**^{**1**} und **R**^{**2**} für Wasserstoff stehen und
die Reste **R**^{**3**}gleich sind und aus der Gruppe enthaltend Wasserstoff, Fluor, C₁-C₁₀-Alkyl oder CF₃ ausgewählt werden,
die Reste **R**^{**4**} gleich sind und aus der Gruppe enthaltend Wasserstoff, Fluor, C₁-C₁₀-Alkyl oder CF₃ ausgewählt werden,
**Y** für einen zweibindigen Rest ausgewählt aus der Gruppe enthaltend CR⁹₂, CHR⁹, (cis)-CH=CH, CR⁹₂CR¹⁰₂, CHR⁹CHR¹⁰, 1,2-arylen, CHR⁹-O-CHR¹⁰ oder CR⁹₂-O-CR¹⁰₂ steht,
wobei **R**^{**9**} und **R**^{**10**} gleich oder verschieden sein und ansonsten unabhängig voneinander ausgewählt werden aus der Gruppe enthaltend Wasserstoff; **Q**; einfach, mehrfach oder unsubstituierte C₁₋C₁₀-Alkyle, C₃-C₁₀-Cycloalkyle, C₂-C₁₀-Alkenyle, C₄-C₁₀₋Cycloalkenyle, C₂-C₁₀-Alkinyle, C₆-C₁₅-Aryle, und C₁-C₁₅₋Heteroaryle, wobei gegebenenfalls die Substituenten ihrerseits die Bedeutung von **Q** haben können und
**Q** ausgewählt wird aus der Gruppe enthaltend -F, -Cl, -Br, -I, -CN, -NO₂, -NR⁷R⁸, -NR⁷OR⁸, -OR⁷, -C(O)R⁷, SR⁷, -SO₃R⁷, -C(O)OR⁷, -C(O)NR⁷R⁸, -OC(O)R⁷, -NR⁷C(O)R⁸,
**R**^{**7**} und **R**^{**8**} gleich oder verschieden sein und ansonsten unabhängig voneinander die Bedeutung von R⁹ haben können,
**R**^{**5**} und **R**^{**6**} gleich oder verschieden sein können und unabhängig voneinander ausgewählt werden aus der Gruppe enthaltend einfach, mehrfach oder unsubstituierte C₃-C₁₀-Cycloalkyle, C₄-C₁₀₋Cycloalkenyle, C₅-C₁₅-Aryle, oder C₁-C₁₅-Heteroaryle, wobei gegebenenfalls die Substituenten ihrerseits die Bedeutung von **Q** haben.

Ein weiterer Gegenstand der Erfindung ist die Verwendung der Verbindungen der allgemeinen Formel (I) als Liganden zur Herstellung von Komplexen enthaltend mindestens einen Liganden der allgemeinen Formel (I) und mindestens ein metallisches oder halbmetallisches Zentrum.
Die mit den erfindungsgemäßen Liganden erhältlichen Katalysatoren zeichnen sich dadurch aus, dass durch ihren Einsatz bei der Synthese chiraler Verbindungen gleichermaßen hohe Produktivitaten, hohe Aktivitäten und hohe Selektivitäten erzielt werden können.

Da die erfindungsgemäßen Biphenylverbindungen der allgemeinen Formel (I) im Gegensatz zu den von *Qiu et al*. bekannten Ligandensystemen nur eine Drehachse als Chiralitätselement besitzen, sind zu deren Synthese teure chirale Edukte entbehrlich und bei deren Synthese kommt es nicht zur Bildung von Diastereomerengemischen, deren Trennung einen zusätzlichen Prozessschritt notwendig machen und die mögliche Ausbeute an reinen Isomeren vermindert.
Die Trennung der bei der Herstellung der erfindungsgemäßen Liganden der allgemeinen Formel (I) erhältlichen Racemate enthaltend Enantiomerenpaare ist dagegen ohne besonderen apparativen Aufwand, beispielsweise mittels einfacher Cokristallisation, möglich.

Durch die Ringgröße (durch Variation von **Y**) und Substitution (durch Variation von **R**^{**3**} und **R**^{**4**}) der Dioxacyclen kann die Verdrillung der Biphenylachse gesteuert und somit der sog. Bisswinkel der erfindungsgemäßen Liganden den jeweiligen Anforderungen entsprechend eingestellt werden. Die erfindungsgemäß 7-bis 9-gliedrigen Ringe am Biphenylgerüst erzeugen durch ihre Nichtplanarität sterische Hinderung in der Ligandensphäre. Somit erfolgt zusätzlich zum Effekt der Axialchiralität eine Verstärkung der chiralen Induktion durch sterische Einflüsse, ohne dass zusätzliche chirale Zentren im Liganden vorhanden sind.

Bevorzugte Reste für R⁹ und R¹⁰ bzw. R⁷ und R⁸ aus der Gruppe der C₁-C₁₀-Alkyle werden ausgewählt aus der Gruppe enthaltend Methyl, Ethyl, n-Propyl, iso-Propyl, n-Butyl, iso-Butyl oder tert-Butyl.

Bevorzugte Reste für R⁹ und R¹⁰ bzw. R⁷ und R⁸ aus der Gruppe C₃₋C₁₀-Cycloalkyle werden ausgewählt aus der Gruppe enthaltend cyclo-Propyl, cyclo-Butyl, cyclo-Pentyl oder cyclo-Hexyl.

Bevorzugte Reste für R⁹ und R¹⁰ bzw. R⁷ und R⁸ aus der Gruppe C₂₋C₁₀-Alkenyle werden ausgewählt aus der Gruppe enthaltend Vinyl, iso-Propenyl oder 2-Methyl-2-butenyl.

Bevorzugte Reste für R⁹ und R¹⁰ bzw. R⁷ und R⁸ aus der Gruppe C₄₋C₁₀-Cycloalkenyle werden ausgewählt aus der Gruppe enthaltend cyclo-Pent-2-enyl, cyclo-Pent-3-enyl, cyclo-Hex-1-enyl, cyclo-Hex-2-enyl oder cyclo-Hex-3-enyl.

Bevorzugte Reste für R⁹ und R¹⁰ bzw. R⁷ und R⁸ aus der Gruppe C₂₋C₁₀-Alkinyle werden ausgewählt aus der Gruppe enthaltend Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 1-Hexinyl, 2-Hexinyl, 3-Hexinyl, 4-Hexinyl oder 5-Hexinyl.

Bevorzugte Reste für R⁹ und R¹⁰ bzw. R⁷ und R⁸ aus der Gruppe C₆₋C₁₅-Aryle werden ausgewählt aus der Gruppe enthaltend Phenyl, Naphthyl oder Anthracenyl.

Bevorzugte Reste für R⁹ und R¹⁰ bzw. R⁷ und R⁸ aus der Gruppe C₁₋C₁₅-Heteroaryle werden ausgewählt aus der Gruppe enthaltend Pyrrolyl, Imidazolyl, Furanyl, Pyridyl, Pyrimidyl, Pyrazolyl, Indolyl, Benzimidazolyl, Benzofuranyl, Oxazolyl, Thiophenyl, Thiazolyl oder Benzothiazolyl.

Bevorzugte Reste für Q werden ausgewählt aus der Gruppe enthaltend -F, -Cl, -Br, -I, -CN, -NO₂ , N(Me)₂, N(Et)₂, N(Pr)₂, N(iso-Pr)₂, NHOMe, N(Me)OMe, N(Et)OEt, N(Me)OEt, OMe, OEt, Oiso-Pr, OBn, C(O)Me, C(O)Et, C(O)CF₃, C(O)Ph, SMe, SEt, SPh, SBn, SO₃Me, SO₃Et, SO₃Ph, C(O)OMe, C(O)OEt, C(O)OPh, C(O)OBn, C(O)N(Me)₂, C(O)N(Et)₂, C(O)NHMe, C(O)NH₂, C(O)N(isoPr)₂, C(O)NHEt, C(O)NH(isoPr), C(O)NHMe, C(O)NH(nPr), C(O)N(nPr)₂, C(O)NHBu, C(O)N(Bu)₂, C(O)NHBn, OC(O)Me, OC(O)Et, OC(O)CF₃, OC(O)Ph, NHC(O)Me, NHC(O)Et, NHC(O)CF₃, NMeC(O)Me, NMeC(O)Et oder NHC(O)Ph, insbesondere F, Cl, CN, NO₂, NMe₂, NEt₂, NHOMe, OMe, OEt, Oiso-Pr, OBn, C(O)Me, C(O)CF₃, SMe, C(O)OMe, C(O)N(Me)₂, C(O)NHMe, OC(O)Me, OC(O)CF₃, NHC(O)Me oder NHC(O)CF₃.

Bevorzugt stehen die Reste R⁵ und R⁶ für ein mit Q substituiertes oder unsubstituiertes Phenyl bzw. Cyclohexyl. In einer besonders bevorzugten Ausführungsform der erfindungsgemäßen Liganden der allgemeinen Formel (I) sind die Reste R⁵ und R⁶ gleich und werden aus den vorgenannten bevorzugten Ausführungsformen ausgewählt.

Besonders bevorzugt handelt es sich bei R⁵ und R⁶ um einen Phenylsubstituenten.

Weiterhin werden die Reste R⁹ und R¹⁰ bevorzugt ausgewählt aus der Gruppe enthaltend Wasserstoff, Methyl, Ethyl, Propyl, Fluor, oder CF₃. In einer besonders bevorzugten Ausführungsform der erfindungsgemäßen Liganden der allgemeinen Formel (I) sind die Reste R⁹ und R¹⁰ gleich und werden aus den vorgenannten bevorzugten Ausführungsformen ausgewählt.

In einer alternativen Ausführungsform, in der Y für CR⁹₂ steht, bildet Y insgesamt einen Spirosubstituenten, wobei C ein quartäres Kohlenstoff-Atom ist und R⁹ ausgewählt wird aus der Gruppe enthaltend (CH₂)₂, (CH₂)₃ oder (CH₂)₄.

In typischen Ausführungsformen der erfindungsgemäßen Liganden ist R³=R⁴=H, wobei Y ausgewählt wird aus der Gruppe enthaltend CH₂, (CH₂)₂, C (CH₃)₂, 1, 2-arylen, CH=CH, CH₂OCH₂ oder (CF₂)₂. Alternativ können R³=R⁴=F sein, wobei Y für (CF₂)₂ steht, oder sind R³=R⁴=CH₃, wobei Y gleich (CH₂)₂ ist.

Mögliche konkrete Ausführungsformen für erfindungsgemäße Verbindungen bzw. Liganden der allgemeinen Formel (I) sind im Folgenden dargestellt.

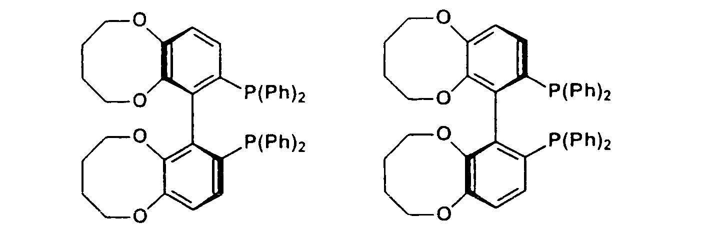

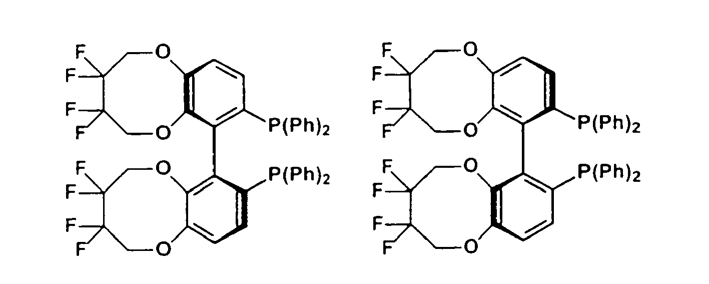

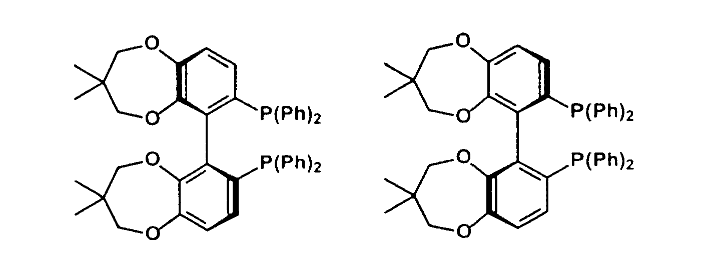

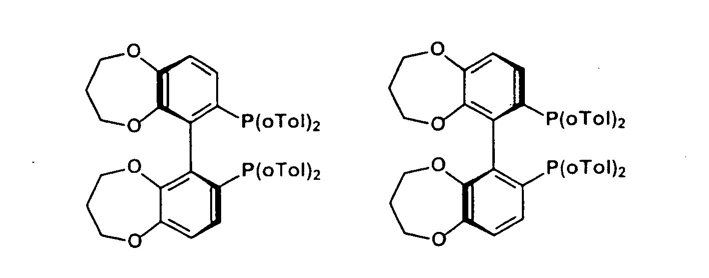

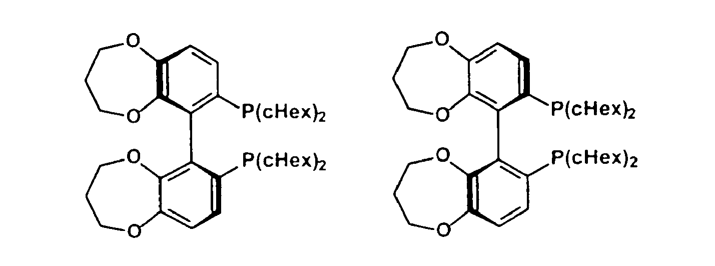

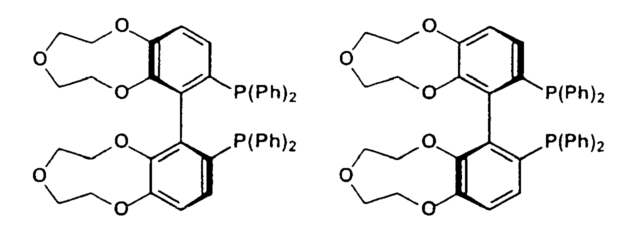

Eine besonders bevorzugte Ausführungsform der erfindungsgemäßen Verbindungen bzw. Liganden der allgemeinen Formel (I) sind (*S*)-(-)-[6,6'-Bis-(3,4-Dihydro-2*H*-1,5-benzodioxepin)-7,7'-diyl]bis-(diphenylphosphin) **(VIIa)** 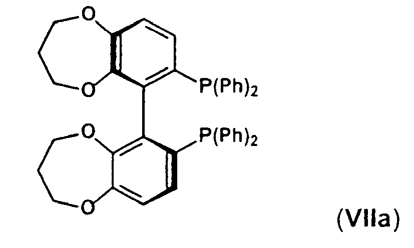 und (*R*)-(+)-[6,6'-Bis-(3,4-Dihydro-2*H*-1,5-benzodioxepin)-7,7'-diyl]bis(diphenylphosphin) (**VIIb**)

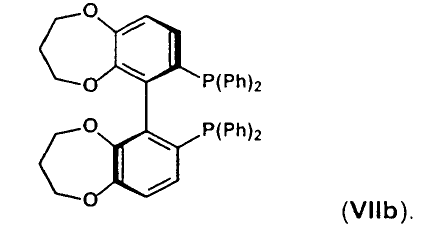

Die Herstellung der erfindungsgemäßen Verbindungen kann durch dem Fachmann bekannte Reaktionsschritte nach den im Schema 1 wiedergegebenen Verfahrensschritten leicht durchgeführt werden. Ein auf den konkreten Einzelfall übertragbares Syntheseprinzip für Verbindungen der allgemeinen Formel (I) wird insbesondere in den Beispielen veranschaulicht.

Ein allgemeines Syntheseprinzip für Liganden vom Biaryldiphosphintyp ist dem Fachmann insbesondere aus Genet, J.-P. et al. Organic Process Research & Development, **2003**, 7, S. 399-406 und Saito, T. et al. Adv. Synth. Catal. **2001**, 345(3), S. 264-267 bekannt.

Die in Schema 1 an den Reaktionspfeilen angegebenen arabischen Ziffern beziehen sich auf die im Folgenden im Einzelnen beschriebenen Verfahrenschritte:

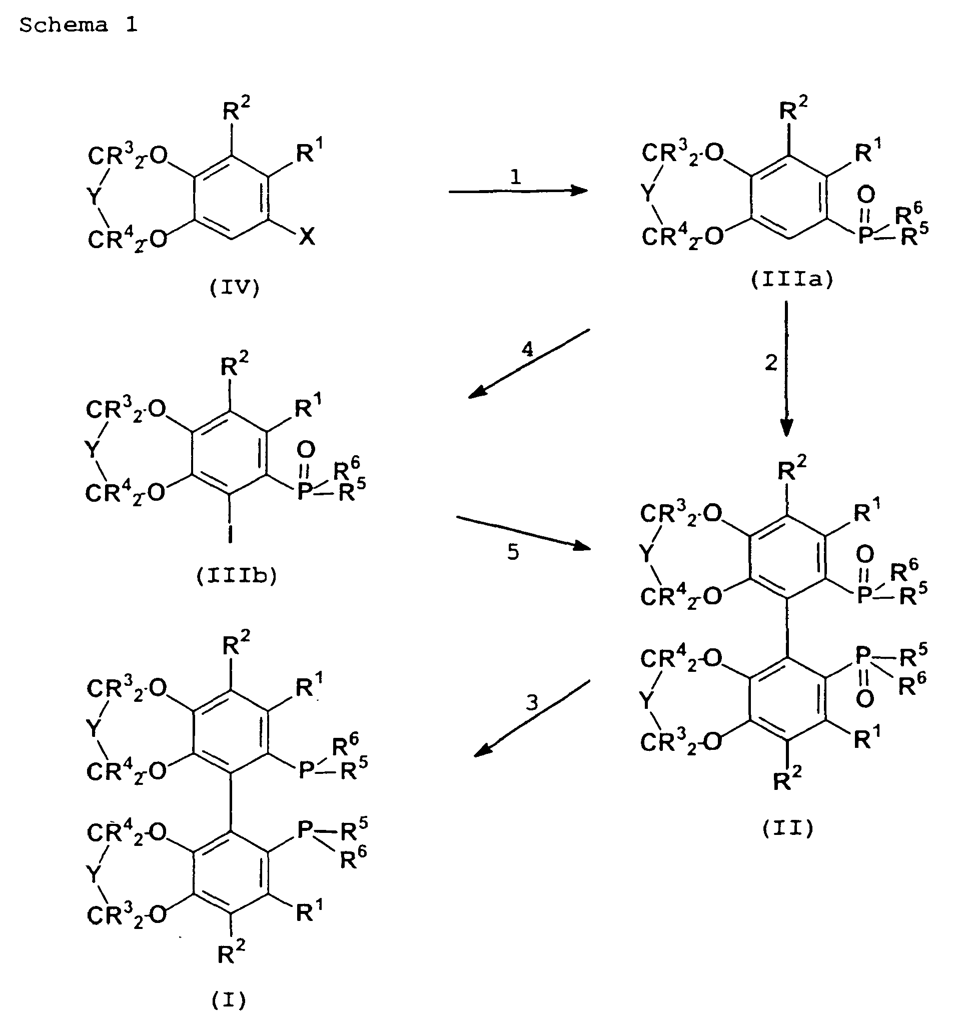

Die erfindungsgemäßen Verbindungen der allgemeinen Formel (I) sowie die Verbindungen der allgemeinen Formel (II) können in enantiomerenreiner, enantiomerenangereicherter oder racemischer Form dargestellt werden.

Die Verbindungen der Formel (I) können in ihren optisch angereicherten Formen (*R*) oder (*S*) oder in ihren racemischen Formen durch Reduktion von Verbindungen der Formel (II) erhalten werden (**3**, Schema 1), 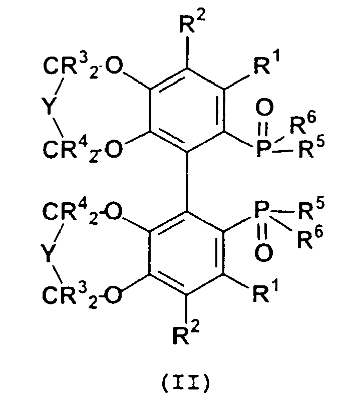 wobei für Verbindungen der allgemeinen Formel (II) R¹ bis R⁶ die oben für die Verbindungen der allgemeinen Formel (I) angegebenen Bedeutungen aufweisen, und (I) und (II) für eine optisch reine oder optisch angereicherte (R)- oder (S)-Form oder die racemische Form stehen können (Ia, Ib bzw. IIa bzw. IIb).

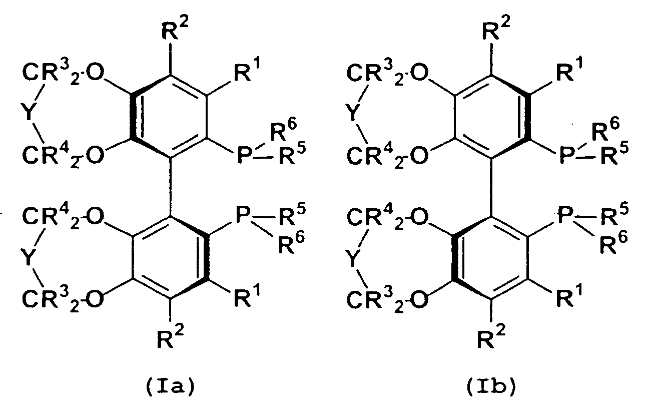

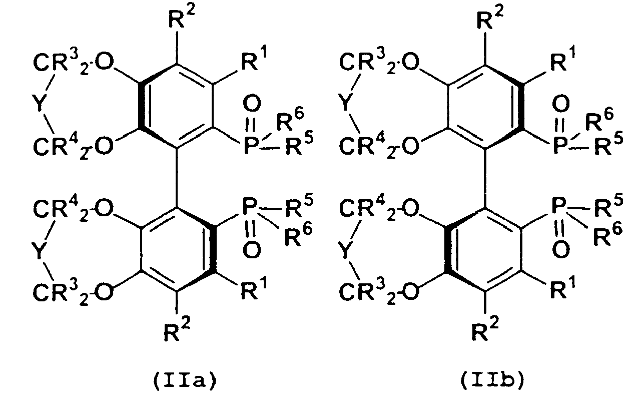

Die Verbindungen der Formel (II), die in racemischer, enantiomerenreiner oder enantiomerenangereicherter Form Intermediate darstellen, sind ebenfalls Gegenstand der vorliegenden Erfindung, wobei R¹ bis R¹⁰, Y und Q die oben für die Verbindungen der allgemeinen Formel (I) angegebenen Bedeutungen aufweisen, insbesondere den oben genannten bevorzugten Ausführungsformen entsprechen.

Die Reduktion wird in einer möglichen Ausführungsform durch Einwirken eines reduzierenden Stoffes, bevorzugt von Trichlorsilan, in Gegenwart eines Amins, bevorzugt von Dimethylanilin, durchgeführt (**3**, Schema 1). Das verallgemeinerbare Prinzip wird in Beispiel 4 veranschaulicht.

Die Verbindungen der Formel (II) werden in enantiomerenreiner oder enantiomerenangereicherter Form beispielsweise durch die Spaltung von racemischem (II) durch Kristallisation in Gegenwart komplexbildender chiraler Verbindungen erhalten. Eine bevorzugte Vorgehensweise ist die Komplexbildung mit chiralen Säuren durch fraktionierte Kristallisation, insbesondere mit (-)-L-Dibenzoylweinsäure oder (+)-D-Dibenzoylweinsäure, die dem Fachmann aus dem Stand der Technik, insbesondere aus Noyori, R. et al. in J. Org. Chem. **1986**, 51, 629ff für diese Art der Racematspaltung geeignet erscheinen. Eine verallgemeinerbare Vorgehensweise wird in Beispiel 3 veranschaulicht.

Alternativ können die Enantiomeren beispielsweise durch chromatographische Trennung erhalten werden.

Alternativ kann eine Trennung in die Enantiomere auch anhand der Verbindungen der allgemeinen Formel (I) erfolgen, insbesondere über chirale Pd-Komplexe, wie dem Fachmann aus Noyori, R. et al. in J. Am. Chem. Soc. **1980**, 102, S. 7932ff bekannt ist.

Die Verbindungen der Formel (II) wiederum können aus Verbindungen der allgemeinen Formel (IIIa), 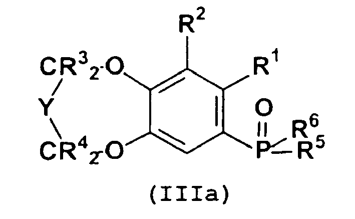 wobei R¹ bis R¹⁰, Y und Q die oben für die Verbindungen der allgemeinen Formel (I) angegebenen Bedeutungen aufweisen, beispielsweise durch oxidative Kupplung, bevorzugt durch Einwirken von Lithiumorganylen, besonders bevorzugt Lithiumdiisopropylamid, in Gegenwart eines geeigneten Oxidationsmittels, bevorzugt von Eisen(III)chlorid, hergestellt werden (**2**, Schema 1). Eine verallgemeinerbare Synthesevorschrift wird in Beispiel 2 veranschaulicht.

Alternativ können die Verbindungen der Formel (II) ebenfalls aus Derivaten (IIIa) in zwei Schritten (**4/5**, Schema 1) hergestellt werden:
a) Iodierung der Verbindung (IIIa), bevorzugt durch Deprotonierung mit Lithiumdiisopropylamid und anschließendes Einwirken von 1,2-Diiodethan, zu iodierten Derivaten der Formel (IIIb) (**4**, Schema 1), 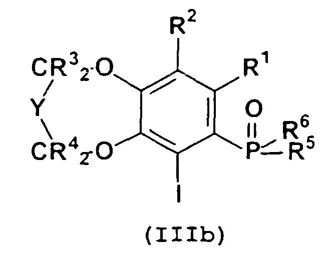
b) gefolgt von einer durch ein Metall katalysierten Kupplungsreaktion, bevorzugt einer Kupfer-katalysierten Kupplungsreaktion (**5**, Schema 1), zu Verbindungen der Formel (II).

Die Verbindungen (IIIa) können aus Verbindungen der allgemeinen Formel (IV), wobei X für Halogen, bevorzugt für Brom, steht und R¹ bis R⁴ die oben für die Verbindungen der allgemeinen Formel (I) angegebenen Bedeutungen aufweisen, bevorzugt über die entsprechenden Grignard-Spezies und anschließende Reaktion mit einem Phosphinylchlorid R⁵R⁶P (O) Cl, wobei R⁵ und R⁶ die oben für (I) angegebenen Bedeutungen aufweisen, hergestellt werden (**1**, Schema 1).

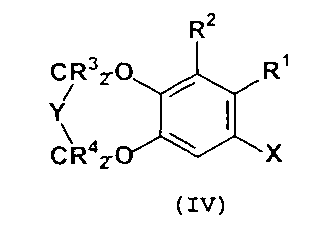

Ein weiterer Gegenstand der Erfindung sind die synthetisch wertvollen Zwischenprodukte zur Herstellung der Verbindungen der allgemeinen Formel (I).

So sind ein weiterer Gegenstand der Erfindung Verbindungen der allgemeinen Formel (IIIa) oder (IIIb) 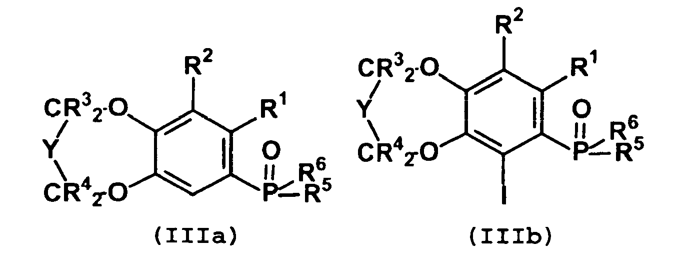 wobei R¹ bis R¹⁰, Y und Q die oben für die Verbindungen der allgemeinen Formel (I) angegebenen Bedeutungen aufweisen, insbesondere den oben genannten bevorzugten Ausführungsformen entsprechen.

In bevorzugte Ausführungsformen für Verbindungen der allgemeinen Formel (IIIa) oder (IIIb) steht CR³₂-Y-CR⁴₂ für (CH₂)₃ und sind R¹=R²=H und R⁵=R⁶=Ph.

Verbindungen der allgemeinen Formel (IV) sind Edukte für die Synthese der erfindungsgemäßen Liganden 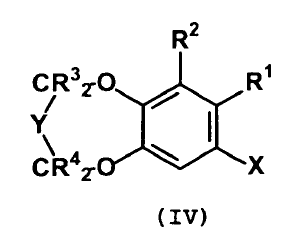 wobei R¹ bis R⁴, R⁷ bis R¹⁰, Q, X und Y die oben angegebenen Bedeutungen aufweisen, insbesondere den oben genannten bevorzugten Ausführungsformen entsprechen, verbunden mit der Maßgabe, dass gleichzeitig CR³₂-Y-CR⁴₂ nicht für (CH₂)₃ und X nicht für Br (R¹ und R² stehen per Definition für Wasserstoff) stehen dürfen.

Die Verbindungen der allgemeinen Formel (IV) als Edukte für die Synthese der erfindungsgemäßen Liganden sind im speziellen Fall, in dem gleichzeitig CR³₂-Y-CR⁴₂ für (CH₂)₃ und X für Br (R¹ und R² stehen per Definition für Wasserstoff) stehen kommerziell verfügbar, die anderen Vertreter der Verbindungsklasse können durch eine einfacheverallgemeinerbare Synthesefolge hergestellt werden, die hier für das kommerziell verfügbare Edukt dargestellt ist:

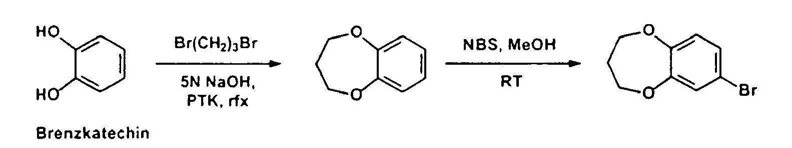

Die Synthesefolge hinsichtlich des Ringsystems ist dem Fachmann insbesondere aus Eynde, J. J. V. et al. Synthetic Communications, **2001**, 31(1), S. 1-7 bekannt.

Ein weiterer Gegenstand der Erfindung ist die Verwendung der Verbindungen der allgemeinen Formel (I) als Liganden zur Herstellung von Komplexen enthaltend mindestens einen Liganden der allgemeinen Formel (I) und mindestens ein metallisches Zentrum.

Die Liganden können in racemischer, enantiomerenreiner oder enatiomerenangereicherter Form eingesetzt und an ein metallisches Zentrum koordiniert werden.

Ein weiterer Gegenstand der Erfindung sind demnach Metallkomplexe enthaltend mindestens einen Liganden der allgemeinen Formel (I) und mindestens ein metallisches Zentrum.

Neben den Liganden der allgemeinen Formel (I) können optional weitere ionische oder neutrale Liganden anwesend sein.

Die erfindungsgemäßen Metallkomplexe können ganz allgemein als homogene oder in immobilisierter Form als heterogene Katalysatoren in der organischen Synthese eingesetzt werden.

Das metallische Zentrum kann allgemein ausgewählt werden aus der Gruppe enthaltend neutral oder ionisch vorliegende Hauptgruppenmetalle, oder den neutral oder ionisch vorliegenden Metallen der Nebengruppenelemente des PSE. Bevorzugt sind insbesondere solche Metalle als metallische Zentren, die ihrer allgemeinen chemischen Natur nach und unter Berücksichtigung ihrer Oxidationsstufe zur Bildung von Phosphinkomplexen geeignet erscheinen. Insbesondere wird der einschlägige Fachmann solche Metalle auswählen, die nach dem allgemeinen Fachwissen für den bestimmten zu katalysierenden Reaktionstyp als typische Katalysemetalle gelten.

Die Koordinations- und Katalyse-Eigenschaften der erfindungsgemäßen Komplexe enthaltend die erfindungsgemäßen Liganden können durch die Wahl der Substituenten am Biphenylgerüst den jeweiligen Anforderungen entsprechend eingestellt werden. Neben der Möglichkeit der Substitution des Biphenylgerüstes durch verschieden große Ringe, mit oder ohne Substituenten, durch die der Koordinationswinkel (sog. "Bisswinkel") des Liganden im Komplex variiert werden kann, wird durch die Möglichkeit von Substitutionen am gesamten Liganden sowie der Variation der Reste R⁵ und R⁶ die Möglichkeit gegeben, die sterischen und elektronischen Verhältnisse und damit letztlich die Koordinationseigenschaften dieser Verbindungen den notwendigen Gegebenheiten gezielt anzupassen. So können beispielsweise durch elektronenziehende, insbesondere wenn R³ für F steht, oder e-lektronenschiebende Substituenten, insbesondere wenn R⁵ und R⁶ für Cyclohexyl stehen, die Koordinationseigenschaften der Phosphoratome den jeweiligen Anforderungen entsprechend eingestellt werden.

In einer bevorzugten Ausführungsform der erfindungsgemäßen Komplexe werden die Verbindungen der allgemeinen Formel (I) in enantiomerenangereicherter oder, besonders bevorzugt, in enantiomerenreiner Form als Ligand verwendet und an ein Metall, insbesondere ein Übergangsmetall komplexiert, wobei chirale Komplexe resultieren.

Wird als Ligand eine Verbindung der allgemeinen Formel (I) in racemischer Form verwendet, kann die Chiralität des Metallkomplexes auch mittels eines weiteren, chiralen Liganden, bevorzugt mit Hilfe eines koordinierten chiralen Diamins erreicht werden. Alternativ kann auch gleichzeitig ein enantiomerenreiner Ligand in Form einer Verbindung der allgemeinen Formel (I) und ein chirales Diamin als weiterer Ligand zugegen sein.

Ein besonders bevorzugtes chirales Diamin ist (S,S)- oder (R,R)-1,2-Diphenylethylendiamin.

Mögliche Ausführungsformen für solche Komplexe sind Ru-Komplexe mit rac-VII, VIIa oder VIIb in Verbindung mit (S,S)- oder (R,R)-1,2-Diphenylethylendiamin, insbesondere [Ru (*rac*-[6,6'-Bis-(3,4-Dihydro-2*H*-1,5-benzodioxepin)-7,7'-diyl]bis(diphenylphosphin))Cl₂ (S,S)-1,2-Diphenylethylendiamin] = [Ru (*rac*-**VII**)Cl₂ (S,S)-1,2-Diphenylethylendiamin] bzw. [Ru ((*R*)-(+)-[6,6'-Bis-(3,4-Dihydro-2*H*-1,5-benzodioxepin)-7,7'-diyl]bis(diphenylphosphin))Cl₂ (S,S)-1,2-Diphenylethylendiamin] = [Ru **(VIIb)** Cl₂ (S,S)-1,2-Diphenylethylendiamin].

Die erfindungsgemäßen Metallkomplexe können allgemein als Katalysatoren in der organischen Synthese, bevorzugt in Form der erfindungsgemäßen chiralen Metallkomplexe als chirale Katalysatoren in der asymmetrischen organischen Synthese verwendet werden.

Allgemein eignen sich die Katalysatoren für Hydrierungs-, Isomerisierungs- und C-C-Verknüpfungsreaktionen.

Die erfindungsgemäßen chiralen Katalysatoren können für asymmetrische Synthesen, bevorzugt für die asymmetrische Hydrierung von ungesättigten Verbindungen, die Isomerisierung von Olefinen und für asymmetrische C-C-Bindungsknüpfungs-Reaktionen eingesetzt werden.

Besonders bevorzugt finden die erfindungsgemäßen Katalysatoren, die Liganden der allgemeinen Formel (I) enthalten, Anwendung bei der Hydrierung von C=O, C=C oder C=N Gruppen. Die für diese Reaktion üblicherweise verwendeten Katalysatoren basieren bevorzugt auf Rhodium, Ruthenium, Iridium, Palladium, Kupfer oder Nickel.

Eine mögliche Ausführungsform für erfindungsgemäße Metallkomplexe sind Verbindungen der allgemeinen Formel (V)

MₘLᵣXₚS_{q} (V)

wobei
**M** für ein Metall ausgewählt aus der Gruppe enthaltend Rhodium, Ruthenium, Iridium, Palladium oder Nickel und
**L** eine Verbindung der allgemeinen Formel (I) darstellt
und im übrigen
**X, S, m, r, p** und **q** wie folgt definiert sind:
   für M = Rh ist X = Cl, Br, I; m = r = p = 2; q = 0;
   für M = Ru ist X = -OC (O) CH₃ (OAc) ; m = r = 1; p = 2; q = 0;
      oder X = Br; m = r = 1; p = 2; q = 0;
      oder X = Cl; m = r = 1; p = 2; q = 0;
      oder X = Cl; S = N(CH₂CH₃)₃;m = r = 2; p = 4;q = 1;
      oder X = methylallyl; m = r = 1; p = 2; q = 0;
      oder X = Cl; S = Pyridin; m = r = 1; p = q = 2;
      oder X = Cl; S= ein chirales 1,2-Diamin; m = r = 1; p = q = 2;
      oder X = Cl; S= ein chirales 1,2-Diamin; m = r = 1; p = 2; q= 1;
   für M = Ir ist X = Cl, Br, oder I; m = r = p = 2; q = 0;
   für M = Pd ist X = Cl; m = r = 1; p = 2; q = 0;
      oder X = π-allyl; m = r = p = 2; q = 0;
   für M = Ni ist X = Cl, Br oder I; m = r = 1; p = 2; q = 0.

Eine weitere mögliche Ausführungsform der erfindungsgemäßen Metallkomplexe sind Verbindungen der allgemeinen Formel (VI)

[M_{w}LₛZₜWᵤ]Aᵥ (VI)

wobei
**M** für ein Metall ausgewählt aus der Gruppe enthaltend Rhodium, Ruthenium, Iridium, Palladium oder Kupfer steht und
**L** eine Verbindung der allgemeinen Formel (I) darstellt
und im übrigen
**Z**, **W, A, w, s, t, u** und **v** wie folgt definiert sind:
   für M = Rh ist Z = 1,5-Cyclooctadien (cod) oder Norbornadien nbd); A = BF₄, ClO₄, PF₆, OTf oder BPh₄;
      w = s = t = v = 1; u = 0;
   für M = Ru ist Z = Cl, Br oder I; W = Benzol oder p-Cymol;
      A = Cl, Br oder I;
      w = s = t = u = v = 1;
   oder A = BF₄, ClO₄, PF₆, BPh₄; w = s = 1; t = u = 0;
      v = 2;
   oder Z = Cl; A = NH₂(C₂H₅)₂; w = s = 2; t = 5; u = 0;
      v = 1;
   für M = Ir ist Z = cod oder nbd; A = BF₄, ClO₄, PF₆ oder BPh₄;
      w = s = v = t = 1, u = 0;
   für M = Pd ist A = BF₄, ClO₄, PF₆ oder BPh₄; w = s = v = 1;
      t = u = 0;
   für M = Cu ist A = ClO₄, PF₆; w = s = v = 1; t = u = 0.

In besonders bevorzugten Ausführungsformen der erfindungsgemäßen Komplexe der allgemeinen Formeln (V) und (VI) wird M ausgewählt aus der Gruppe enthaltend Rhodium, Ruthenium oder Iridium. Weiter wird in solchen besonders bevorzugten Ausführungsformen ein Ligand **L** der allgemeinen Formel (I) in einer enantiomerenreinen Form eingesetzt, insbesondere ausgewählt aus den oben genannten besonders bevorzugten Ausführungsformen für Verbindungen der allgemeinen Formel (I).

Die erfindungsgemäßen Komplexe, insbesondere die vorgenannten Verbindungen der allgemeinen Formeln (V) und (VI) können allgemein durch in der Literatur beschriebene bzw. dem Fachmann bekannte Verfahren hergestellt werden, insbesondere gemäß den in Mashima, K. et al. J. Org. Chem. **1994**, 59, S. 3064-3076; Genet, J.-P. et al. Tetrahedron Lett., 36(27), **1995**, S. 4801-4804; King, S. A. et al. J. Org. Chem. **1992**, 57, S. 6689-6691; Ager, D. J. et al. Tetrahedron: Asymmetry, 8(20), S. 3327-3355, **1997** beschrieben bzw. den dort zitierten Verfahren.

Die erfindungsgemäßen Komplexe werden im allgemeinen aus einem sog. Metallkomplex-Precursor hergestellt, dessen Natur vom ausgewählten Metall abhängt. Als Precursoren kommen typischerweise [Rh(cod)₂]OTf, [Rh(cod)₂]BF₄, [Rh(cod)₂]ClO₄, [Rh(cod)₂]BPh₄, [Rh(cod)₂]PF₆, [Rh(nbd)₂]OTf, [Rh(nbd)₂]BF₄, [Rh(nbd)₂]ClO₄, [Rh(nbd)₂]BPh₄, [Rh(nbd)₂]PF₆, [{Rh(cod)}₂(µ-Cl)₂], RuCl₃, [RuCl₂(Benzol)]₂, [RuCl₂(cod)]ₙ, [{RuBr (p-Cymol)}₂(µ-Br)₂], [{RuI (p-Cymol)}₂(µ-I)₂], [{RuCl (p-Cymol)}₂(µ-Cl)₂], [Ir(cod)₂]OTf, [Ir(cod)₂]BF₄, [Ir(cod₂]ClO₄, [Ir(cod)₂]BPh₄, [Ir(cod)₂]PF₆, [Ir(nbd)₂]OTf, [Ir(nbd)₂]BF₄, [Ir(nbd)₂]ClO₄, [Ir(nbd)₂]BPh₄, [Ir(nbd)₂]PF₆, [{Ir(cod)}₂(µ-Cl)₂], [Ir(cod) (CH₃CN)₂]BF₄, Pd(OAc)₂, PdCl₂, PdBr₂, [PdCl₂(CH₃CN)₂], [PdCl₂(cod)], [Pd(π-allyl)Cl]₂, [Pd(methylallyl)Cl]₂, [Pd(CH₃CN)₄(BF₄)₂], NiCl₂, NiBr₂, NiI₂, Cu(acac)₂, Cu(ClO₄)₂, CuCl, CuBr, oder CuI in Frage.

Die erfindungsgemäßen Metall-Komplexe werden im allgemeinen durch Mischen des Metallkomplex-Precursors mit dem Liganden der allgemeinen Formel (I) in einem geeigneten, gegebenenfalls wasserfreien und entgasten organischen Lösemittel hergestellt (vgl. Beispiele 6 und 7). Die Reaktionstemperatur kann dabei zwischen 15 und 150 °C, bevorzugt zwischen 30 und 120 °C, betragen.

Als geeignete Lösemittel kommen alle dem Fachmann für diese Reaktion bekannten Lösemittel, insbesondere aromatische Kohlenwasserstoffe wie z. B. Benzol, Toluol; Amide wie z. B. Dimethylformamid, N-Methylpyrrolidinon; chlorierte Kohlenwasserstoffe wie z.B. Dichlormethan, Trichlormethan; Alkohole wie z. B. Methanol, Ethanol, n- bzw. iso-Propanol; Ketone wie z. B. Aceton, Methylethylketon, Cyclohexanon; Ether wie z. B. Tetrahydrofuran, Diethylether, Methyltertbutylether; lineare, verzweigte und cyclische Alkane wie z. B. Pentan, Hexan, Cyclohexan und Mischungen der vorgenannten Lösemittel in Frage.

Die Komplexe können sowohl nach dem Fachmann bekannten Methoden isoliert als auch ohne vorausgehende Isolierung *in situ* eingesetzt werden.

Ein weiterer Gegenstand der Erfindung ist die Verwendung der erfindungsgemäßen Metallkomplexe als Katalysatoren in der organischen Synthese, bevorzugt als chirale Katalysatoren in asymmetrischen Reaktionen. Diese katalytischen Verfahren können auf dem Fachmann bekannte Weise durchgeführt werden.

So wird beispielsweise im Falle der asymmetrischen Hydrierung eine Lösung des ungesättigten Substrats zusammen mit dem Metall-Katalysator unter Einwirkung von Wasserstoff oder einem Hydriddonor, beispielsweise einem Alkohol, umgesetzt. Die Reaktionsbedingungen bei diesem Verfahren sind analog den aus der Literatur oder dem Fachmann bekannten Bedingungen (z.B.: Ager, D. J.; Laneman, S. A. in Tetrahedron: Asymmetry, Vol. 8, 20, S. 3327-3355, **1997**; bzw. Tang, W.; Zhang, X. in Chem. Rev., 103, S. 3029-3069, **2003**, sowie darin zitierte Literaturstellen).

So kann der Wasserstoffdruck in einem Bereich zwischen 1 und 150 bar, bevorzugt in einem Bereich zwischen 1 und 50 bar, liegen. Die Temperatur kann in einem Bereich zwischen 0 und 150 °C, bevorzugt in einem Bereich zwischen 20 und 100 °C, liegen. Das molare Substrat/Katalysator (S/C) -Verhältnis kann in einem Bereich zwischen 100 : 1 und 250 000 : 1, bevorzugt in einem Bereich zwischen 300 : 1 und 20 000 : 1, liegen.

Dem Substrat können zur Hydrierung weitere Stoffe, wie beispielsweise Salze oder Säuren, zugesetzt werden. Bevorzugt können organische Säuren, deren Salze oder anorganische Säuren oder deren Salze zugesetzt werden. Besonders bevorzugt können Methansulfonsäure, Trifluormethansulfonsäure, para-Toluolsulfonsäure, Essigsäure, Trifluoressigsäure, Salzsäure, Schwefelsäure oder deren Salze zugesetzt werden.

Eine bevorzugte Verwendung der erfindungsgemäßen Katalysatoren ist die asymmetrische Hydrierung von Doppelbindungen, ausgewählt aus der Gruppe enthaltend C=C, C=O und C=N.

In einer typischen Ausführungsform eines erfindungsgemäßen katalytischen Verfahrens unter Verwendung der erfindungsgemäßen Metallkomplexe enthaltend den erfindungsgemäßen Liganden der allgemeinen Formel (I) wird eine methanolische Lösung von 3-Oxo-pentansäuremethylester (50 Gew.-%) in Gegenwart von [RuCl(p-Cymol)(**VIIb**)]Cl (0.05 Mol-%) und Methansulfonsäure (0.025 Mol-%) bei 100 °C und 10 bar Wasserstoffdruck 24 Stunden lang gerührt. Nach destillativer Reinigung kann als Produkt der enantioselektiven Carbonylhydrierung (*R*)-3-Hydroxypentansäure-methylester in hoher optischer (>98 %ee) und chemischer Reinheit (>98%) erhalten werden.

Eine besonders bevorzugte Verwendung der erfindungsgemäßen Katalysatoren ist die asymmetrische Hydrierung von Carbonylverbindungen.

Die erfindungsgemäßen Diphosphinliganden der allgemeinen Formel (I) und deren Metallkomplexe ermöglichen die Herstellung von chiralen Verbindungen in hohen Ausbeuten und mit hohen optischen Reinheiten.

Der Aufbau der erfindungsgemäßen Diarylverbindungen ist ohne Einsatz teurer chiraler Reagenzien möglich und leicht durchführbar. Durch die Variation der Ringgrößen und -substitutionen am Biarylgerüst kann der Torsionswinkels des Liganden, je nach Anwendungsbedarf, eingestellt werden.

Es konnte gezeigt werden, dass die erfindungsgemäßen axialchiralen Diphosphinliganden zur Erreichung hoher Enantioselektivitäten bei asymmetrischen Umsetzungen befähigt sind, ohne dass dazu zusätzliche Stereozentren im Liganden vorhanden sein müssen, welche den Syntheseaufwand deutlich erhöhen würden.

Die aus dem Stand der Technik bekannten Diphosphinliganden und deren Anwendungen geben dem Fachmann keinerlei Hinweise auf die unerwartet hohe Leistungsfähigkeit der erfindungsgemäßen Diphosphinliganden, die auf die zusätzlichen bei Qui et al. beschrieben Merkmale verzichten.

Die folgenden Beispiele dienen der Veranschaulichung der vorliegenden Erfindung.

### Beispiel 1

### Synthese von 3,4-Dihydro-2H-1,5-benzodioxepin-7-diphenylphosphinoxid (im folgenden DBO genannt)

In einem 1L Dreihalskolben mit Magnetrührung, Rückflußkühler, Innenthermometer und Tropftrichter wurden unter Argonatmosphäre 6.05 g (248 mmol) Magnesiumspäne in 280 mL Tetrahydrofuran (THF) vorgelegt. Unter Rühren wurde eine Lösung von 55 g (240 mmol) 7-Brom-3,4-dihydro-2*H*-1,5-benzodioxepin in 14 mL THF innerhalb 60 min. zugetropft und die Temperatur der Mischung im Bereich von 60-70 °C gehalten. Nach 3 Stunden Rühren wurde die Lösung auf 0 °C gekühlt und 39.2 mL (205 mmol) Diphenylphosphinylchlorid innerhalb 90 min. zugetropft, wobei die Temperatur zwischen 0 und 10 °C gehalten wurde. Anschließend wurde 15 Stunden bei Raumtemperatur gerührt.
Bei ca. 10 °C wurden erst 62 mL Wasser, dann 72 mL 1N HCl langsam zugegeben und anschließend 90 min. gerührt. Nach Verdünnen mit 240 mL Wasser wurde die Lösung mit Methylenchlorid (3 x 200 mL) extrahiert, die organischen Phasen vereinigt und nacheinander mit 1N HCl (240 mL), gesättigter aq NaHCO₃-Lösung (240 mL), Wasser (240 mL) und gesättigter aq NaCl-Lösung (240 mL) gewaschen. Nach Trocknen über Na₂SO₄ wurde das Lösungsmittel im Vakuum entfernt und der Rückstand bei 60 °C im Vakuum getrocknet. Nach Umkristallisieren aus 200 mL Toluol erhielt man 68.8 g (196 mmol) 3,4-Dihydro-2*H*-1,5-benzodioxepin-7-diphenylphosphinoxid als gelblichen Feststoff.
Schmelzpunkt: 147-149 °C
1H-NMR (CDCl₃, 500 MHz), δ=2.21 (m, 2H), 4.22 (t, 2H), 4.29 (t, 2H), 7.02 (s, 1H), 7.17-7.28 (m, 2H), 7.42-7.49 (m, 4H), 7.53 (t, 2H), 7.67 ppm (q, 4H).
31P-NMR (CDCl₃, 121 MHz), δ= 28.8 ppm.

### Beispiel 2

### Synthese von (±) - [6, 6' -Bis- (3, 4-Dihydro-2H-1, 5-benzodioxepin) - 7,7'-diyl]bis(diphenylphosphinoxid) (im folgenden (±)-Bis-DBO genannt) - Kupplung

In einem 2L Vierhalskolben mit KPG-Rührer, Innenthermometer, Tropftrichter und Argoneinlaß wurden unter Argonatmosphäre 18.4 mL (120 mmol) Diisopropylamin in 95 mL THF vorgelegt und bei - 78 bis -65 °C innerhalb von 60 min. mit 70 mL n-Buthyllithium-Lösung (1.6N in Hexan, 106 mmol) versetzt. Nach vollständiger Zugabe ließ man die Mischung bis -10 °C erwärmen und kühlte dann auf -70°C. Innerhalb von 4 Stunden gab man eine Lösung von 35 g (100 mmol) 3,4-Dihydro-2*H*-1,5-benzodioxepin-7-diphenylphosphinoxid (DBO) in 880 mL THF zu während die Temperatur bei -70°C gehalten wurde. Nach vollständiger Zugabe ließ man die Mischung innerhalb 30 min. auf -40 °C erwärmen, kühlte anschließend auf -78 °C und gab innerhalb 30 min. eine Lösung von 16.2 g (100 mmol) Eisen(III)chlorid in 140 mL THF zu wobei man die Temperatur unter -65 °C hielt. Nach vollständiger Zugabe wurde die Mischung für 15 Stunden bei Raumtemperatur gerührt. Nach Entfernen des Lösungsmittels im Vakuum wurde der Rückstand in 700 mL Methylenchlorid aufgenommen und nacheinander mit 10 %-iger aq HCl (350 mL), Wasser (350 mL) und gesättigter aq NaCl-Lösung (350 mL) gewaschen. Nach Trocknen über Na₂SO₄ wurde das Lösungsmittel im Vakuum entfernt und der Rückstand aus Methylenchlorid / Ethylacetat umkristallisiert. Man erhielt 17.1 g (25 mmol) (±)-[6,6'-Bis-(3,4-Dihydro-2*H*-1,5-benzodioxepin)-7,7'-diyl]bis(diphenylphosphinoxid) als schmutzig-weißen Feststoff.
Schmelzpunkt: 259-261 °C.
1H-NMR (CDCl₃, 500 MHz), δ= 1. 69 (m, 2H) , 1. 95 (m, 2H) , 3. 69 (m, 4H), 4.00 (m, 2H), 4.21 (m, 2H), 6.70-6.83 (m, 4H), 7.24-7.31 (m, 4H), 7.33-7.42 (m, 6H), 7.45 (m, 2H), 7.57 (dd, 7.6, 12.2 Hz, 4H), 7.62 ppm (dd, 7.6, 11.4 Hz, 4H).
31P-NMR (CDCl₃, 121 MHz), δ= 29.4 ppm.

### Beispiel 3

### Racemattrennung von (±) - [6, 6' -Bis- (3, 4-Dihydro-2H-1, 5-benzodioxepin)-7,7'-diyl]bis(diphenylphosphinoxid) - Herstellung von (+)-Bis-DBO und (-)-Bis-DBO

Zu einer zum Rückfluss erhitzten Lösung von 5.59 g (8 mmol) (±)-[6,6'-Bis-(3,4-Dihydro-2*H*-1,5-benzodioxepin)-7,7'-diyl]bis-(diphenylphosphinoxid) in 50 mL Dichlormethan wurde unter Rühren eine Lösung von 1.43 g (4 mmol) (-)-Dibenzoylweinsäure in 25 mL Ethylacetat zugegeben. Nach zwei Stunden erhitzen unter Rückfluss wurde die Mischung auf Raumtemperatur abgekühlt, der Niederschlag abgetrennt und im Vakuum getrocknet (Auswage 2.55 g). Die Mutterlauge wurde eingedampft und getrennt behandelt (s.u.).
Der abgetrennte Niederschlag (2.55 g) wurde in 25 mL Dichlormethan aufgenommen, mit 15 mL aq. NaOH (2N) versetzt und 2 Stunden gerührt. Nach Abtrennen der Wasserphase wurde die organische Phase mit 2 x 20 mL aq. NaOH (2N) und anschließend mit ges. NaCl-Lösung gewaschen, über Na₂SO₄ getrocknet und das Lösemittel im Vakuum entfernt. Man erhielt 1.72 g (-)-Bis-DBO als farblosen Feststoff:
[α]^{D}₂₀= -96.8° (c= 1g/100mL CHCl₃)
HPLC (Hexan/Isopropanol = 92/8; Flußrate: 1 mL/min): 99.8 %ee (44.617 min), >98 % chemische Reinheit.

Die eingedampfte Mutterlauge (4.54 g) wurde in 40 mL Dichlormethan aufgenommen, mit 20 mL aq. NaOH (2N) versetzt und 1 Stunde gerührt. Nach Abtrennen der Wasserphase wurde die organische Phase mit 2 x 10 mL aq. NaOH (2N) und anschließend mit ges. NaCl-Lösung gewaschen, über Na₂SO₄ getrocknet und das Lösemittel im Vakuum entfernt. Man erhielt 4.31 g Feststoff, der in 40 mL Dichlormethan gelöst wurde und unter Erhitzen unter Rückfluss mit einer Lösung von 1.43 g (4 mmol) (+)-Dibenzoylweinsäure in 25 mL Ethylacetat versetzt und für 2 Stunden unter Rückfluss erhitzt wurde. Nach Abkühlen der Mischung auf Raumtemperatur wurde der Niederschlag abgetrennt und im Vakuum getrocknet (Auswage 3.42 g), in 40 mL Dichlormethan aufgenommen, mit 20 mL aq. NaOH (2N) versetzt und 1 Stunden gerührt. Nach Abtrennen der Wasserphase wurde die organische Phase mit 2 x 20 mL aq. NaOH (2N) und anschließend mit ges. NaCl-Lösung gewaschen, über Na₂SO₄ getrocknet und das Lösemittel im Vakuum entfernt. Man erhielt 2.21 g (+)-Bis-DBO als farblosen Feststoff:
[α]^{D}₂₀= +96.8° (c= 1g/100mL CHCl₃)
HPLC (Hexan/Isopropanol = 92/8; Flussrate: 1 mL/min): 99.8%ee (35.424 min), >98 % chemische Reinheit.

### Beispiel 4

Synthese von (*S*)-(-)-[6,6'-Bis-(3,4-Dihydro-2*H*-1,5-benzodioxepin)-7,7'-diyl]bis(diphenylphosphin) **VIIa** - Reduktion

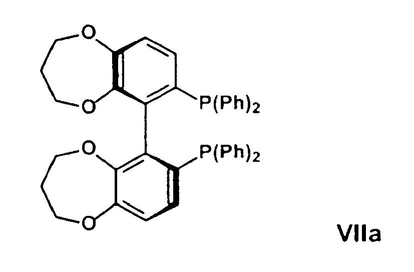

In einem 100 mL Dreihalskolben mit Magnetrührung, Rückflußkühler, Innenthermometer und Tropftrichter wurden 1.4 g (2 mmol) (-)-[6,6'-Bis-(3,4-Dihydro-2*H*-1,5-benzodioxepin)-7,7'-diyl]bis-(diphenylphosphinoxid), 35 mL Toluol und 2.9 mL (21.9 mmol) N,N-Dimethylanilin vorgelegt und unter starkem Rühren mit 2.1 mL (20 mmol) Trichlorsilan versetzt. Nach 8 Stunden Rühren bei 100 °C wurde die Mischung abgekühlt und bei 0 °C vorsichtig mit 25 mL 4N aq NaOH versetzt und für 1 Stunde bei Raumtemperatur gerührt. Die organische Phase wurde abgetrennt und die wässrige Phase mit Toluol (2 x 20 mL) extrahiert. Die vereinigten organischen Phasen wurden nacheinander mit 1N aq HCl (3 x 50 mL), Wasser (50 mL) und gesättigter aq NaCl-Lösung (50 mL) gewaschen und nach Trocknen über Na₂SO₄ wurde das Lösungsmittel im Vakuum entfernt, der Rückstand in 50 mL Dichlormethan aufgenommen, im Vakuum eingedampft und aus Dichlormethan / Methanol umkristallisiert. Man erhielt 1.02 g (-)-[6,6'-Bis-(3,4-Dihydro-2*H*-1,5-benzodioxepin)-7,7'-diyl]bis(diphenylphosphin) **(VIIa)** als farblose Nadeln.
Schmelzpunkt: 247-249 °C
1H-NMR (CDCl₃, 500 MHz), δ=1.67 (m, 2H), 1.94 (m, 2H), 3. 30 (m, 2H), 3.66-3.82 (m, 4H), 4.17 (m, 2H), 6.66 (d, 8.1 Hz, 2H), 6.87 (d, 8.1 Hz, 2H), 7.16-7.26 ppm (m, 20H).
31P-NMR (CDCl₃, 121 MHz), δ= -15.4 ppm.
[α]^{D}₂₀= -25.1° (c= 1g/100mL CHCl₃)
HPLC (Daicel "Chiracel OD-H";Hexan/Isopropanol = 98/2; Flussrate: 0.5 mL/min; 40 °C): >99 %ee (10.442 min), >99 % chemische Reinheit.

### Beispiel 5

### Synthese von (R)-(+)-[6,6'-Bis-(3,4-Dihydro-2H-1,5-benzodioxepin)-7,7'-diyl]bis(diphenylphosphin) VIIb

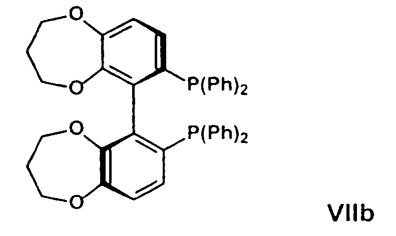

Analog Beispiel 4 aus 2.1 g (+)-[6,6'-Bis-(3,4-Dihydro-2*H*-1,5-benzodioxepin)-7,7'-diyl]bis(diphenylphosphinoxid). Man erhielt 1.41 g (+)-[6,6'-Bis-(3,4-Dihydro-2*H*-1,5-benzodioxepin)-7,7'-diyl]bis(diphenylphosphin) (**VIIb**) als kristallinen Feststoff. Schmelzpunkt: 248-249 °C
1H-NMR (CDCl₃, 500 MHz), δ=1.67 (m, 2H), 1.94 (m, 2H), 3. 30 (m, 2H), 3.66-3.82 (m, 4H), 4.17 (m, 2H), 6.66 (d, 8.1 Hz, 2H), 6.87 (d, 8.1 Hz, 2H), 7.16-7.26 ppm (m, 20H).
31P-NMR (CDCl₃, 121 MHz), δ= -15.4 ppm.
[α]^{D}₂₀= +25.1° (c= 1g/100mL CHCl₃)
HPLC (Daicel "Chiracel OD-H";Hexan/Isopropanol = 98/2; Flußrate: 0.5 mL/min; 40 °C): >99 %ee (9.200 min), > 99 % chemische Reinheit.

### Beispiel 6

### Synthese von [RuCl(p-Cymol)(VIIa)]Cl

In einem 50 mL Schlenkkolben mit Magnetrührung und Rückflußkühler wurden unter Argonatmosphäre 100 mg (0.15 mmol) (-)-[6,6'-Bis-(3,4-Dihydro-2*H*-1,5-benzodioxepin)-7,7'-diyl]bis-(diphenylphosphin) **(VIIa)** in 11 mL Methylenchlorid gelöst, mit 51 mg (0.083 mmol) [{RuCl(p-Cymol)}₂(µ-Cl)₂] und 4 mL Methanol versetzt und für eine Stunde bei 50 °C gerührt. Die klare orange-farbene Lösung wurde anschließend im Vakuum eingedampft und der Rückstand im Hochvakuum getrocknet. Man erhielt 143 mg (0.147 mmol) des rotbraunen Ru-Komplexes.

### Beispiel 7:

### Synthese von [RuCl(p-Cymol)(VIIb)]Cl

Die Synthese wurde mit 100 mg (0.15 mmol) (+)-[6,6'-Bis-(3,4-Dihydro-2*H*-1,5-benzodioxepin)-7,7'-diyl]bis(diphenylphosphin) (**VIIb**) analog Beispiel 6 ausgeführt. Man erhielt 145 mg (0.149 mmol) des Ru-Komplexes als rotbraunen Feststoff.

### Beispiel 8:

### Hydrierung von 3-Oxo-pentansäuremethylester mit [RuCl(p-Cymol) (VIIb)]Cl

### (S/C = 2000)

Eine Lösung von 3.8 mL Methanol und 3 g (23 mmol) 3-Oxo-pentansäuremethylester wurde unter Argonatmosphäre und Ultraschallbestrahlung für 20 min. entgast. Nach Zugabe von 11.2 mg (0.0115 mmol) [RuCl(p-Cymol)(**VIIb**)]Cl und 0.55 mg (0.0058 mmol) Methansulfonsäure wurde die Lösung in einem Stahlautoklaven mit Glaseinsatz für 24 Stunden bei 100 °C unter 10 bar Wasserstoffdruck kräftig gerührt. Nach Abkühlen auf Raumtemperatur wurde die Mischung destillativ gereinigt. Man erhielt 3.04 g (*R*)-3-Hydroxypentansäuremethylester.
99.9 %ee (GC); >99% chemische Reinheit.

### Beispiel 9:

### Hydrierung von 3-Oxo-pentansäuremethylester mit [RuCl(p-Cymol) (VIIa) ]Cl

### (s/c = 2000)

Eine Lösung von 3.8 mL Methanol und 3 g (23 mmol) 3-Oxo-pentansäuremethylester wurde mit [RuCl(p-Cymol)(**VIIa)**]Cl in der für Beispiel 8 beschriebenen Weise behandelt. Man erhielt 3.03 g (S)-3-Hydroxypentansäuremethylester.
99.9 %ee (GC); >99% chemische Reinheit.

### Beispiel 10:

### Synthese von [RuCl(Benzol)(VIIa)]Cl

In einem 50 mL Schlenkkolben mit Magnetrührung und Rückflußkühler wurden unter Argonatmosphäre 100 mg (0.15 mmol) **VIIa** in 11 mL Methylenchlorid gelöst, mit 42 mg (0.083 mmol)
[{RuCl(Benzol)}₂(µ-Cl)₂] und 4 mL Methanol versetzt und für eine Stunde bei 50 °C gerührt. Die klare orange-farbene Lösung wurde anschließend im Vakuum eingedampft und der Rückstand im Hochvakuum getrocknet. Man erhielt 143 mg (0.147 mmol) des rotbraunen Ru-Komplexes.

### Beispiel 11:

### Hydrierung von 3-Oxo-pentansäuremethylester mit [RuCl(Benzol)(VIIa)]Cl

### (s/c = 2000)

Eine Lösung von 3.8 mL Methanol und 3 g (23 mmol) 3-Oxo-pentansäuremethylester wurde mit 0.0115 mmol
[RuCl(Benzol)**(VIIa)**]Cl in der für Beispiel 8 beschriebenen weise behandelt. Man erhielt 3.03 g (*S*)-3-Hydroxypentansäure-methylester.
99.6 %ee (GC); >99% chemische Reinheit.

### Beispiel 12:

### Synthese von [RuBr₂(VIIa)]

In einem 10 mL Rundkolben mit Magnetrührung wurden unter Argonatmosphäre 4.7 mg (0.007 mmol) **VIIa** und 2.4 mg (0.0075 mmol) Bis-(Methallyl)-cyclooctadien-Ruthenium(II) in 1 mL Aceton vorgelegt, mit 16 µl (0.014 mmol) methanolischer HBr-Lösung (48 Gew.-%) versetzt und für 30 min. bei Raumtemperatur gerührt. Die braune Lösung wurde anschließend im Vakuum eingedampft und der Rückstand im Hochvakuum getrocknet. Der so erhaltene rotbraunen Ru-Komplexes wurde anschließend zur Hydrierung eingesetzt.

### Beispiel 13:

### Hydrierung von 3-Oxo-pentansäuremethylester mit [RuBr₂(VIIa)]

### (s/c = 2000)

Eine Lösung von 3.8 mL Methanol und 3 g (23 mmol) 3-Oxo-pentansäuremethylester wurde mit 0.0115 mmol [RuBr₂**(VIIa)**] in der für Beispiel 8 beschriebenen Weise behandelt. Man erhielt 2.88 g (*S*)-3-Hydroxypentansäuremethylester.
99.5 %ee (GC); 95% chemische Reinheit.

### Beispiel 14:

### Asymmetrische Michael-Addition an 2-Cyclohexen-1-on

Ein Gemisch aus 0,039 mmol (*S*)-(-)-[6,6'-Bis-(3,4-Dihydro-2*H-*1,5-benzodioxepin)-7,7'-diyl]bis(diphenylphosphin) (**VIIa**), 9,95 mg Rh (acac) (C₂H₄)₂, 6, 42 mmol Phenylboronsäure, 4 ml Dioxan, 0,4 mL Wasser und 1,3 mmol 2-Cyclohexen-1-on wurde unter Argonatmosphäre für 6 Stunden bei 100 °C gerührt. Nach Abkühlen auf Raumtemperatur wurde im Vakuum eingedampft, der Rückstand in 50 mL Ethylacetat aufgenommen und die organische Phase nach Waschen mit 20 mL gesättigter wässriger NatriumhydrogencarbonatLösung über Magnesiumsulfat getrocknet. Nach Entfernen des Lösemittels im Vakuum wurde der Rückstand an Kieselgel gereinigt. Man erhielt (S)-3-Phenylcyclohexanon mit einer optischen Reinheit von 97,4 %ee.

### Beispiel 15:

### Asymmetrische Isomerisierung von Diethylgeranylamin

Eine Lösung von 0,025 mmol [Rh (**VIIb**) (COD)]ClO₄ in 5 mL Tetrahydrofuran wurde unter einer Wasserstoffatmosphäre von 1 bar für 20 min. bei Raumtemperatur gerührt. Anschließend wurde 0,52 g (2,5 mmol) (*E*)-*N,N-*Diethyl-3,7-dimethyl-2,6-octadienylamin zugegeben und die Mischung unter Argonatmosphäre bei 40 °C für 24 Stunden gerührt. Nach Entfernen des Lösungsmittels im Vakuum wurde der Rückstand mittels Kugelrohrdestillation gereinigt. Man erhielt (3*S*)-(*E*)-*N*,*N*-Diethyl-3,7-dimethyl-1,6-octadienylamin mit einer optischen Reinheit von 96.8 %ee.

### Beispiel 16:

### Synthese von 3,3,4,4-Tetrafluoro-[2,5H]-1,6-benzodioxocin

In einem 2 **L** Dreihalskolben mit Magnetrührer und Rückflußkühler wurden 31.2 g Brenzkatechin und 39.6 g Kaliumhydroxid in 800 mL Acetonitril vorgelegt und unter Rühren auf 75 °C erhitzt. Nach Zutropfen von 120 g 2,2,3,3-Tetrafluorobutan-1,4-bis-trifluormethansulfonat in 500 mL Acetonitril ließ man die Mischung auf 25 °C abkühlen und rührte noch 3 Stunden. Nach Filtration und Entfernung des Lösemittels im Vakuum wurde der Rückstand in 300 mL MTBE aufgenommen, mit 1N aq HCl (200 mL) und ges. aq NaCl (200 mL) gewaschen und nach Trocken über Natriumsulfat das Lösemittel im Vakuum entfernt. Der Rückstand wurde an Kieselgel (Eluent: Petrolether/ Ethylacetat 8/1) gereinigt. Man erhielt 48 g 3,3,4,4-Tetrafluoro-[2,5*H*]-1,6-benzodioxocin als farblose Kristalle.

### Beispiel 17:

### Synthese von 8-Bromo-3,3,4,4-Tetrafluoro-[2,5H]-1,6-benzodioxocin

Zu einer bei 110 °C gerührten Mischung aus 500 mL Eisessig, 30 g Kaliumbromid und 30 g 3,3,4,4-Tetrafluoro-[2,5*H*]-1,6-benzodioxocin wurde langsam 102 g Brom, in 100 mL Eisessig gelöst, zugetropft. Anschließend wurde die Wärmezufuhr unterbrochen und die Mischung über Nacht bei Raumtemperatur gerührt. Nach Zugabe von 500 mL Wasser wurde die Mischung mit Dichlormethan extrahiert (3 x 300 mL) und die vereinigten organischen Extrakte mit 1N aq Natriumthiosulfatlösung (250 mL) und anschließend mit ges. aq Natriumcarbonatlösung (250 mL) und Wasser gewaschen. Nach Trocknen (Natriumsulfat) und Entfernen des Lösemittels erhielt man ein gelbbraunes Öl. Nach destillativer Reinigung erhielt man 34 g 8-Bromo-3,3,4,4-Tetrafluoro-[2,5*H*]-1,6-benzodioxocin.

### Beispiel 18:

### Synthese von Diphenyl(3,3,4,4-tetrafluoro-[2,5H]-1,6-benzodioxocin-8-yl)-phosphinoxid

In einem 250 mL Dreihalskolben mit Magnetrührung, Rückflußkühler, Innenthermometer und Tropftrichter wurden unter Argonatmosphäre 2 g (83 mmol) Magnesiumspäne in 100 mL Tetrahydrofuran (THF) vorgelegt. Unter Rühren wurde eine Lösung von 25 g (80 mmol) 8-Bromo-3,3,4,4-Tetrafluoro-[2,5*H*]-1,6-benzodioxocin in 10 mL THF innerhalb 20 min. zugetropft und die Mischung für 5 Stunden refluxiert. Nach Abkühlen auf 0 °C wurde 13 mL (68 mmol) Diphenylphosphinylchlorid innerhalb 20 min. zugetropft, wobei die Temperatur zwischen unter 10 °C gehalten wurde. Anschließend wurde über Nacht bei Raumtemperatur gerührt.
Bei ca. 10 °C wurden erst 20 mL Wasser, dann 25 mL 1N HCl langsam zugegeben und anschließend 90 min. gerührt. Nach Verdünnen mit 80 mL Wasser wurde die Lösung mit Methylenchlorid (3 x 70 mL) extrahiert, die organischen Phasen vereinigt und nacheinander mit 1N HCl (80 mL), gesättigter aq NaHCO₃-Lösung (80 mL), Wasser (80 mL) und gesättigter aq NaCl-Lösung (80 mL) gewaschen. Nach Trocknen über Na₂SO₄ wurde das Lösungsmittel im Vakuum entfernt. Nach Umkristallisieren aus 100 mL Toluol erhielt man 26 g (60 mmol) Diphenyl(3,3,4,4-tetrafluoro-[2,5*H*]-1,6-benzodioxocin-8-yl)-phosphinoxid.

### Beispiel 19:

### Synthese von (±)-[7,7'-Bis-(3,3,4,4-tetrafluoro-[2,5H]-1,6-benzodioxocin)-8,8'-diyl]bis(diphenylphosphinoxid) (im folgenden (±)-Bis-F4-DBO genannt) - Kupplung

In einem 1L Vierhalskolben mit KPG-Rührer, Innenthermometer, Tropftrichter und Argoneinlaß wurden unter Argonatmosphäre 9.2 mL (60 mmol) Diisopropylamin in 50 mL THF vorgelegt und bei -78 bis -65 °C innerhalb von 30 min. mit 35 mL n-Buthyllithium-Lösung (1.6N in Hexan, 53 mmol) versetzt. Nach vollständiger Zugabe ließ man die Mischung bis -10 °C erwärmen und kühlte dann auf -70°C. Innerhalb von 2 Stunden gab man eine Lösung von 22 g (50 mmol) Diphenyl(3,3,4,4-tetrafluoro-[2,5*H*]-1,6-benzodioxocin-8-yl)-phosphinoxid in 400 mL THF zu während die Temperatur bei -70°C gehalten wurde. Nach vollständiger Zugabe ließ man die Mischung innerhalb 20 min. auf -40 °C erwärmen, kühlte anschließend auf -78 °C und gab innerhalb 20. min. eine Lösung von 8.1 g (50 mmol) Eisen(III)chlorid in 70 mL THF zu, wobei man die Temperatur unter -65 °C hielt. Nach vollständiger Zugabe wurde die Mischung für 18 Stunden bei Raumtemperatur gerührt. Nach Entfernen des Lösungsmittels im Vakuum wurde der Rückstand in 300 mL Methylenchlorid aufgenommen und nacheinander mit 10 %-iger aq HCl (200 mL), Wasser (200 mL) und gesättigter aq NaCl-Lösung (200 mL) gewaschen. Nach Trocknen über Na₂SO₄ wurde das Lösungsmittel im Vakuum entfernt und der Rückstand aus Methylenchlorid / Ethylacetat umkristallisiert. Man erhielt 10.4g (12 mmol) (±)-[7,7'-Bis-(3,3,4,4-tetrafluoro-[2,5*H*]-1,6-benzodioxocin)-8,8'-diyl]bis(diphenylphosphinoxid) als schmutzig-weißen Feststoff.

### Beispiel 20:

### Racemattrennung von (±)-[7,7'-Bis-(3,3,4,4-tetrafluoro-[2,5H]-1,6-benzodioxocin)-8,8'-diyl]bis(diphenylphosphinoxid) - Herstellung von (+)-Bis-F4-DBO und (-)-Bis-F4-DBO

Zu einer zum Rückfluss erhitzten Lösung von 6.96 g (8 mmol) (±)-[7,7'-Bis-(3,3,4,4-tetrafluoro-[2,5*H*]-1,6-benzodioxocin)-8,8'-diyl]bis(diphenylphosphinoxid) in 60 mL Dichlormethan wurde unter Rühren eine Lösung von 2.86 g (8 mmol)
(-)-Dibenzoylweinsäure in 45 mL Ethylacetat zugegeben. Nach zwei Stunden erhitzen unter Rückfluss wurde die Mischung auf Raumtemperatur abgekühlt, der Niederschlag abgetrennt und im Vakuum getrocknet. Die Mutterlauge wurde eingedampft und getrennt behandelt (s. u.).

Der abgetrennte Niederschlag wurde in 30 mL Dichlormethan aufgenommen, mit 30 mL aq. NaOH (2 N) versetzt und 2 Stunden gerührt. Nach Abtrennen der Wasserphase wurde die organische Phase mit 2 x 20 mL aq. NaOH (2 N) und anschließend mit ges. aq NaCl-Lösung gewaschen, über Na₂SO₄ getrocknet und das Lösemittel im Vakuum entfernt. Man erhielt 3.13 g (-)-Bis-F4-DBO als farblosen Feststoff:

Die eingedampfte Mutterlauge wurde in 50 mL Dichlormethan aufgenommen, mit 30 mL aq. NaOH (2 N) versetzt und 1 Stunde gerührt. Nach Abtrennen der Wasserphase wurde die organische Phase mit 2 x 20 mL aq. NaOH (2 N) und anschließend mit ges. aq NaCl-Lösung gewaschen, über Na₂SO₄ getrocknet und das Lösemittel im Vakuum entfernt. Man erhielt einen Feststoff, der in 50 mL Dichlormethan gelöst wurde und unter Erhitzen unter Rückfluss mit einer Lösung von 2.86 g (8 mmol) (+)-Dibenzoylweinsäure in 45 mL Ethylacetat versetzt und für 2 Stunden unter Rückfluss erhitzt wurde. Nach Abkühlen der Mischung auf Raumtemperatur wurde der Niederschlag abgetrennt und im Vakuum getrocknet, in 60 mL Dichlormethan aufgenommen, mit 30 mL aq. NaOH (2 N) versetzt und 1 Stunden gerührt. Nach Abtrennen der Wasserphase wurde die organische Phase mit 2 x 30 mL aq. NaOH (2 N) und anschließend mit ges. aq NaCl-Lösung gewaschen, über Na₂SO₄ getrocknet und das Lösemittel im Vakuum entfernt. Man erhielt 3.21 g (+)-Bis-F4-DBO als farblosen Feststoff:

### Beispiel 21:

### Synthese von (S)-(-)-[7,7'-Bis-(3,3,4,4-tetrafluoro-[2,5H]-1,6-benzodioxocin)-8,8'-diyl]bis(diphenylphosphin) - Reduktion

In einem 250 mL Dreihalskolben mit Magnetrührung, Rückflußkühler, Innenthermometer und Tropftrichter wurden 2.6 g (3 mmol) (-)-[7,7'-Bis-(3,3,4,4-tetrafluoro-[2,5*H*]-1,6-benzodioxocin)-8,8'-diyl]bis(diphenylphosphinoxid), 50 mL Toluol und 4.4 mL (33 mmol) N,N-Dimethylanilin vorgelegt und unter starkem Rühren mit 3.2 mL (30 mmol) Trichlorsilan versetzt. Nach 8 Stunden Rühren bei 100 °C wurde die Mischung abgekühlt und bei 0 °C vorsichtig mit 40 mL 4 N aq NaOH versetzt und für 1 Stunde bei Raumtemperatur gerührt. Die organische Phase wurde abgetrennt und die wässrige Phase mit Toluol (2 x 30 mL) extrahiert. Die vereinigten organischen Phasen wurden nacheinander mit 1 N aq HCl (3 x 70 mL), Wasser (70 mL) und gesättigter aq NaCl-Lösung (70 mL) gewaschen und nach Trocknen über Na₂SO₄ wurde das Lösungsmittel im Vakuum entfernt, der Rückstand in 70 mL Dichlormethan aufgenommen, im Vakuum eingedampft und aus Dichlormethan / Methanol umkristallisiert. Man erhielt 2.14 g (-)-[7,7'-Bis-(3,3,4,4-tetrafluoro-[2,5*H*]-1,6-benzodioxocin)-8,8'-diyl]bis-(diphenylphosphin) als farblose Nadeln.

### Beispiel 22:

### Synthese von (R)-(+)-[7,7'-Bis-(3, 3, 4, 4-tetrafluoro-[2, 5H]-1, 6-benzodioxocin)-8,8'-diyl]bis(diphenylphosphin) - Reduktion

Analog Beispiel 21 aus 2.4 g (+)-[7,7'-Bis-(3,3,4,4-tetrafluoro-[2,5*H*]-1,6-benzodioxocin)-8,8'-diyl]bis-(diphenylphosphinoxid). Man erhielt 1.98 g (*R*)-(+)-[7,7'-Bis-(3,3,4,4-tetrafluoro-[2,5*H*]-1,6-benzodioxocin)-8,8'-diyl]bis-(diphenylphosphin) als kristallinen Feststoff.

### Beispiel 23:

### Synthese von [RuCl(p-Cymol)((-)-[7,7'-Bis-(3,3,4,4-tetrafluoro-[2,5H]-1,6-benzodioxocin)-8,8'-diyl]bis(diphenylphosphin))]Cl

In einem 50 mL Schlenkkolben mit Magnetrührung und Rückflußkühler wurden unter Argonatmosphäre 126 mg (0.15 mmol) (-)-[7,7'-Bis-(3,3,4,4-tetrafluoro-[2,5*H*]-1,6-benzodioxocin)-8,8'-diyl]bis(diphenylphosphin) in 15 mL Methylenchlorid gelöst, mit 51 mg (0.083 mmol) [{RuCl(p-Cymol)}₂(µ-Cl)₂] und 6 mL Methanol versetzt und für eine Stunde bei 50 °C gerührt. Die Lösung wurde anschließend im Vakuum eingedampft und der Rückstand im Hochvakuum getrocknet. Man erhielt 168 mg (0.147 mmol) des rotbraunen Ru-Komplexes.

### Beispiel 24:

### Synthese von [RuCl(p-Cymol)((+)-[7,7'-Bis-(3,3,4,4-tetrafluoro-[2,5H]-1,6-benzodioxocin)-8,8'-diyl]bis(diphenylphosphin))]Cl

Die Synthese wurde mit 126 mg (0.15 mmol) (+)-[7,7'-Bis-(3,3,4,4-tetrafluoro-[2,5*H*]-1,6-benzodioxocin)-8,8'-diyl]bis-(diphenylphosphin) analog Beispiel 23 ausgeführt. Man erhielt 170 mg (0.149 mmol) des Ru-Komplexes als rotbraunen Feststoff.

### Beispiel 25:

### Hydrierung von 3-Oxo-pentansäuremethylester mit [RuCl(p-Cymol) ((+)-[7,7'-Bis-(3,3,4,4-tetrafluoro-[2,5H]-1,6-benzodioxocin)-8,8'-diyl]bis(diphenylphosphin))]C1

### (S/C = 2000)

Eine Lösung von 3.8 mL Methanol und 3 g (23 mmol) 3-Oxo-pentansäuremethylester wurde unter Argonatmosphäre und Ultraschallbestrahlung für 20 min. entgast. Nach Zugabe von 13.2 mg (0.0115 mmol) [RuCl(p-Cymol)((+)-[7,7'-Bis-(3,3,4,4-tetrafluoro-[2,5*H*]-1,6-benzodioxocin)-8,8'-diyl]bis-(diphenylphosphin))]Cl und 0.55 mg (0.0058 mmol) Methansulfonsäure wurde die Lösung in einem Stahlautoklaven mit Glaseinsatz für 24 Stunden bei 100 °C unter 10 bar Wasserstoffdruck kräftig gerührt. Nach Abkühlen auf Raumtemperatur wurde die Mischung destillativ gereinigt. Man erhielt 3.02 g (*R*)-3-Hydroxypentansäuremethylester.
> 97 %ee (GC); > 97% chemische Reinheit.

## Patentansprüche

1. Verbindungen der allgemeinen Formel (I) 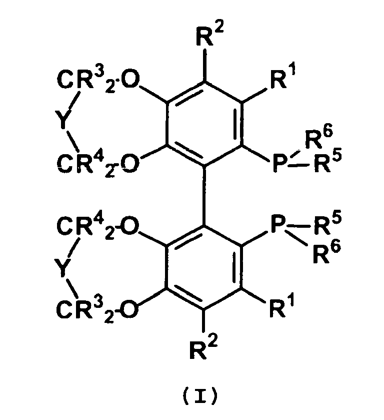 **dadurch gekennzeichnet, dass**
**R**^{**1**} und **R**^{**2**} für Wasserstoff stehen und
die Reste **R**^{**3**} gleich sind und aus der Gruppe enthaltend Wasserstoff, Fluor, C₁-C₁₀-Alkyl oder CF₃ ausgewählt werden,
die Reste **R**^{**4**} gleich sind und aus der Gruppe enthaltend Wasserstoff, Fluor, C₁-C₁₀-Alkyl oder CF₃ ausgewählt werden,
**Y** für einen zweibindingen Rest ausgewählt aus der Gruppe enthaltend CR⁹₂, CHR⁹, (cis)-CH=CH, CR⁹₂CR¹⁰₂, CHR⁹CHR¹⁰, 1,2-arylen, CHR⁹-O-CHR¹⁰ oder CR⁹₂-O-CR¹⁰₂ steht,
wobei **R**^{**9**} und **R**^{**10**} gleich oder verschieden sein und ansonsten unabhängig voneinander ausgewählt werden aus der Gruppe enthaltend Wasserstoff; **Q;** einfach, mehrfach oder unsubstituierte C₁₋C₁₀-Alkyle, C₃-C₁₀-Cycloalkyle, C₂-C₁₀-Alkenyle, C₄-C₁₀₋Cycloalkenyle, C₂-C₁₀-Alkinyle, C₆-C₁₅-Aryle, und C₁-C₁₅₋Heteroaryle, wobei gegebenenfalls die Substituenten ihrerseits die Bedeutung von **Q** haben können und
**Q** ausgewählt wird aus der Gruppe enthaltend -F, -Cl, -Br, -I, -CN, -NO₂, -NR⁷R⁸, -NR⁷OR⁸, -OR⁷, -C(O)R⁷, SR⁷, -SO₃R⁷, -C(O)OR⁷, -C(O)NR⁷R⁸, -OC(O) R⁷, -NR⁷C(O) R⁸,
wobei **R**^{**7**} und **R**^{**8**} gleich oder verschieden sein und ansonsten unabhängig voneinander die Bedeutung von R⁹ haben können,
**R**^{**5**} und **R**^{**6**} gleich oder verschieden sein können und unabhängig voneinander ausgewählt werden aus der Gruppe enthaltend einfach, mehrfach oder unsubstituierte C₃-C₁₀-Cycloalkyle, C₄-C₁₀₋Cycloalkenyle, C₅-C₁₅-Aryle, oder C₁-C₁₅-Heteroaryle, wobei gegebenenfalls die Substituenten ihrerseits die Bedeutung von **Q** haben.

2. Verbindungen nach Anspruch 1 in der optischen Konfiguration der allgemeinen Formeln (Ia) und (Ib)

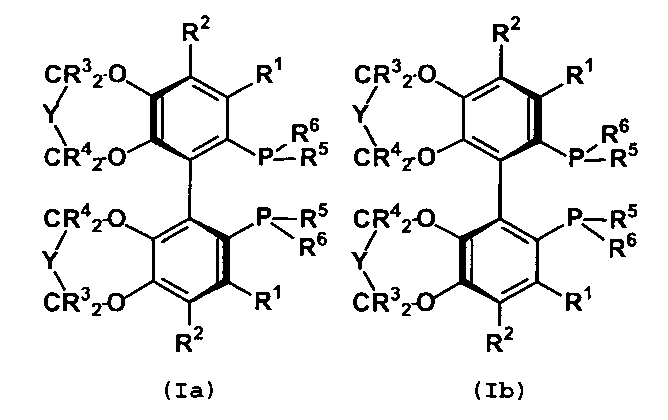

3. Verfahren zur Herstellung von Verbindungen nach Anspruch 1, enthaltend die folgenden Schritte:
A) Umsetzung einer Verbindung der allgemeinen Formel (IV) 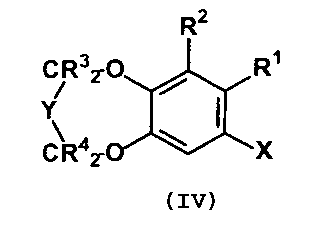 wobei **X** für Halogen steht und
**R**^{**1**}**, R**^{**2**}**, R**^{**3**}**, R**^{**4**}**, R**^{**7**}**, R**^{**8**}**, R**^{**9**}**, R**^{**10**}**, Q** und **Y** die in Anspruch 1 genannten Bedeutungen haben
mit einem Phosphinylchlorid R⁵R⁶P(O)Cl, wobei **R**^{**5**} und **R**^{**6**} die im Anspruch 1 genannten Bedeutungen haben, zu einer Verbindung der allgemeinen Formel (IIIa) 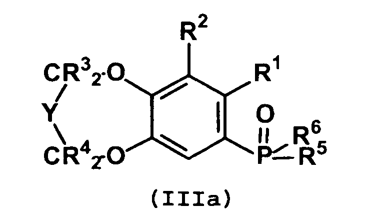
B) Umsetzung der nach Schritt A erhaltenen Verbindung der allgemeinen Formel (IIIa) zu einer Verbindung der allgemeinen Formel (II) 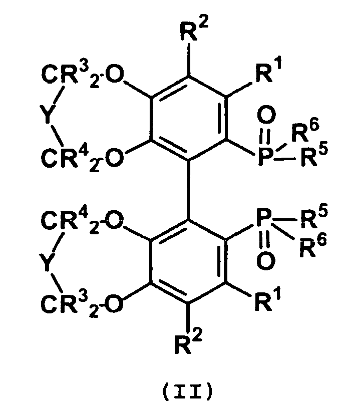 durch oxidative Kupplung und
C) Reduktion der nach Schritt B erhaltenen Verbindung der allgemeinen Formel (II).

4. Verfahren nach Anspruch 3, **dadurch gekennzeichnet, dass** im Schritt B zunächst die aus Schritt A erhaltene Verbindung der allgemeinen Formel (IIIa) durch Iodierung zu einem Zwischenprodukt der allgemeinen Formel (IIIb) 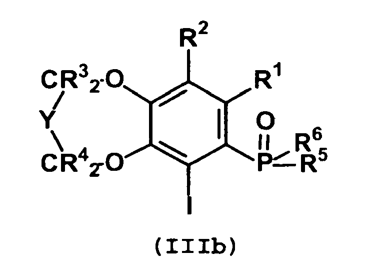 umgesetzt wird und dieses anschließend in einer durch ein Metall katalysierten Kupplungsreaktion in eine Verbindung der allgemeinen Formel (II) gemäß Anspruch 2 überführt wird.

5. Verfahren nach Anspruch 3 oder 4, **dadurch gekennzeichnet, dass** die nach Schritt B erhaltenen Verbindungen der allgemeinen Formel (II) durch fraktionierte Kristallisation in Gegenwart komplexbildender chiraler Verbindungen in enantiomerenreiner oder enantiomerenangereicherter Form der allgemeinen Formeln (IIa) und (IIb) 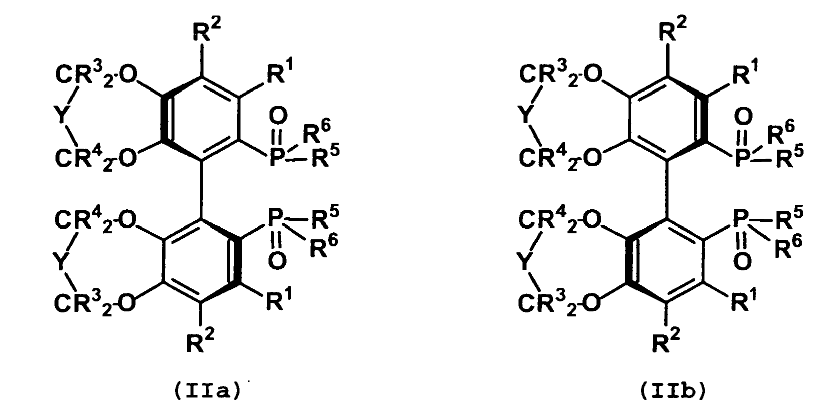 dargestellt werden.

6. Verfahren nach Anspruch 3 oder 4, **dadurch gekennzeichnet, dass** in einem zusätzlichen Schritt D die erhaltenen Verbindungen der allgemeinen Formel (I) mittels Überführung in chirale Palladium-Komplexe in enantiomerenreiner oder enantiomerenangereicherter Form der allgemeinen Formeln (Ia) und (Ib) dargestellt werden.

7. Verbindungen der allgemeinen Formel (II) 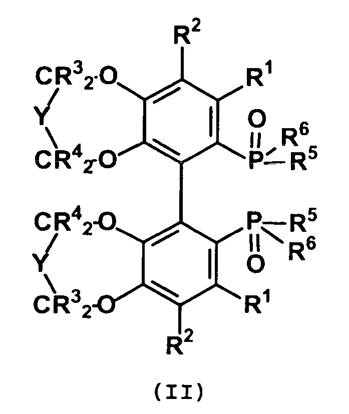 **dadurch gekennzeichnet, dass**
**R**^{**1**}**, R**^{**2**}**, R**^{**3**}**, R**^{**4**}**, R**^{**5**}**, R**^{**6**}**, R**^{**7**}**, R**^{**8**}**, R**^{**9**}**, R**^{**10**}**, Q** und **Y** die in Anspruch 1 genannten Bedeutung haben.

8. Verbindungen nach Anspruch 7 in der optischen Konfiguration der allgemeinen Formeln (IIa) und (IIb)

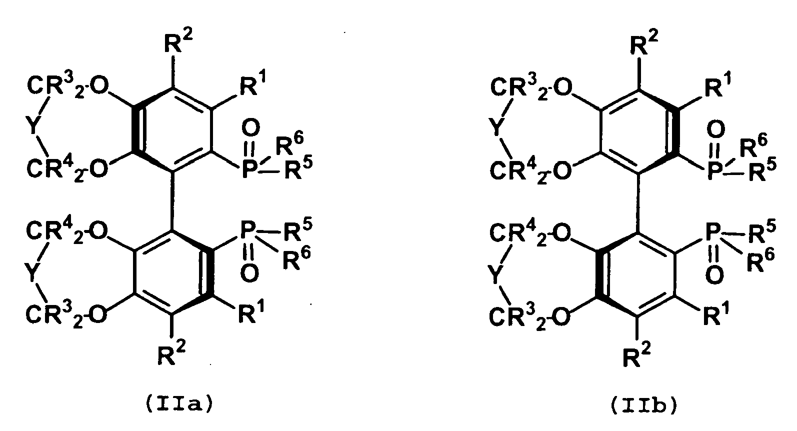

9. Verfahren zur Herstellung enantiomerenreiner oder enatiomerenangereicherter Verbindungen nach Anspruch 8 durch fraktionierte Kristallisation von Verbindungen nach Anspruch 7 in Gegenwart komplexbildender chiraler Verbindungen.

10. Verfahren zur Herstellung enantiomerenreiner oder enatiomerenangereicherter Verbindungen nach Anspruch 2 mittels Überführung von Verbindungen nach Anspruch 1 in chirale Palladium-Komplexe.

11. Verbindungen der allgemeinen Formel (IIIa) oder (IIIb) 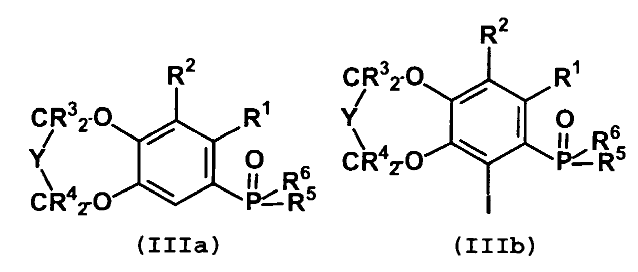 **dadurch gekennzeichnet, dass**
**R**^{**1**}**, R**^{**2**}**, R**^{**3**}**, R**^{**4**}**, R**^{**5**}**, R**^{**6**}**, R**^{**7**}**, R**^{**8**}**, R**^{**9**}**, R**^{**10**}**, Q** und **Y** die in Anspruch 1 genannten Bedeutung haben.

12. Verwendung der Verbindungen der allgemeinen Formel (I) als Liganden zur Herstellung von Komplexen enthaltend mindestens einen Liganden der allgemeinen Formel (I) und mindestens ein metallisches Zentrum.

13. Komplexe enthaltend mindestens einen Liganden der allgemeinen Formel (I) und mindestens ein metallisches Zentrum.

14. Komplexe nach Anspruch 13, **dadurch gekennzeichnet, dass** mindestens ein Ligand der allgemeinen Formel (I) in enantiomerenreiner oder entaiomerenangereicherter Form enthalten ist.

15. Komplexe nach Anspruch 13 oder 14, **dadurch gekennzeichnet, dass** im Falle, dass ein nicht enantiomerenreiner Ligand der allgemeinen Formel (I) enthalten ist, zusätzlich ein anderer chiraler Ligand zugegen ist.

16. Komplexe nach einem oder mehreren der Ansprüche 13 bis 15, **dadurch gekennzeichnet, dass** das metallische Zentrum ausgewählt wird aus der Gruppe enthaltend Rhodium, Ruthenium, Iridium, Palladium, Kupfer oder Nickel.

17. Verfahren zur Herstellung von Komplexen nach Anspruch 13 durch Umsetzung einer Verbindung der allgemeinen Formel (I) mit einem das metallische Zentrum enthaltenden Precursor in Gegenwart eines organischen Lösungsmittels.

18. Verwendung der Komplexe nach einem oder mehreren der Ansprüche 13 bis 16, als Katalysatoren in der organischen Synthese.

19. Verwendung nach Anspruch 18, **dadurch gekennzeichnet, dass** ein chiraler Komplex in der asymmetrischen organischen Synthese als Katalysator eingesetzt wird.

20. Verwendung nach Anspruch 18 oder 19 als homogener oder in immobilisierter Form als heterogener Katalysator in der organischen Synthese.

21. Verwendung nach einem oder mehreren der Ansprüche 18 bis 20 als Katalysator in Hydrierungs-, Isomerisierungs- und C-C-Verknüpfungsreaktionen.

22. Verfahren zur Hydrierung von C=O, C=C oder C=N-Gruppen in einem Substrat, **dadurch gekennzeichnet, dass** die Hydrierung in Gegenwart eines Komplexes nach den Ansprüchen 13 bis 17 durchgeführt wird.

23. Verfahren nach Anspruch 22, **dadurch gekennzeichnet, dass** eine asymmetrischen Hydrierung in Gegenwart eines chiralen Komplexes nach den Ansprüchen 15 oder 16 durchgeführt wird.

24. Verfahren nach Anspruch 23, **dadurch gekennzeichnet, dass** die Hydrierung in Gegenwart eines Komplexes enthaltend (*S*)-(-)-[6,6'-Bis-(3,4-Dihydro-2*H*-1,5-benzodioxepin)-7,7'-diyl]bis-(diphenylphosphin) **(VIIa)** 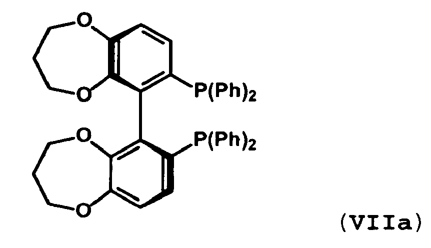 oder (*R*)-(+)-[6,6'-Bis-(3,4-Dihydro-2*H*-1,5-benzodioxepin)-7,7'-diyl]bis(diphenylphosphin) **(VIIb)** 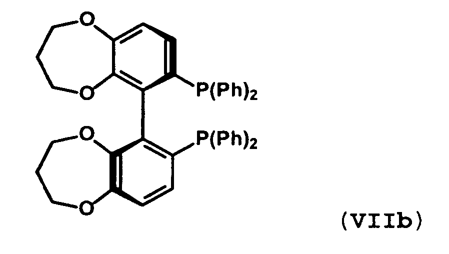 als Liganden durchgeführt wird.

## Claims

1. Compounds of the general formula (I) 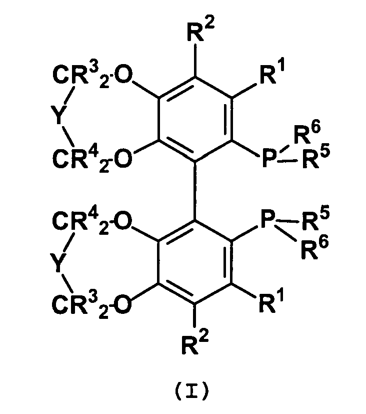 **characterized in that**
R¹ and R² are each hydrogen and
the radicals R³ are identical and are selected from the group consisting of hydrogen, fluorine, C₁-C₁₀-alkyl or CF₃,
the radicals R⁴ are identical and are selected from the group consisting of hydrogen, fluorine, C₁-C₁₀-alkyl or CF₃,
Y is a divalent radical selected from the group consisting of CR⁹₂, CHR⁹, (cis)-CH=CH, CR⁹₂CR¹⁰₂, CHR⁹CHR¹⁰, 1,2-arylene, CHR⁹-O-CHR¹⁰ or CR⁹₂-O-CR¹⁰₂,
where R⁹ and R¹⁰ can be identical or different and otherwise are selected independently from the group consisting of hydrogen; Q; monosubstituted, polysubstituted or unsubstituted C₁-C₁₀-alkyls, C₃-C₁₀₋cycloalkyls, C₂-C₁₀-alkenyls, C₄-C₁₀-cycloalkenyls, C₂₋C₁₀-alkynyls, C₆-C₁₅-aryls and C₁-C₁₅-heteroaryls, where the substituents may in turn have the meanings of Q and
Q is selected from the group consisting of -F, -Cl,-Br, -I, -CN, -NO₂, -NR⁷R⁸, -NR⁷OR⁸, -OR⁷, -C(O)R⁷, SR⁷, -SO₃R⁷, -C(O)OR⁷, - -C(O)NR⁷R⁸, -OC (O)R⁷, -NR⁷C(O)R⁸,
R⁷ and R⁸ can be identical or different and otherwise can independently have the meanings of R⁹,
R⁵ and R⁶ can be identical or different and are selected independently from the group consisting of monosubstituted, polysubstituted or unsubstituted C₃₋C₁₀-cycloalkyls, C₄-C₁₀-cycloalkenyls, C₅-C₁₅-aryls and C₁-C₁₅-heteroaryls, where the substituents may in turn have the meanings of Q.

2. Compounds according to claim 1 in the optical configuration of the general formulae (Ia) and (Ib)

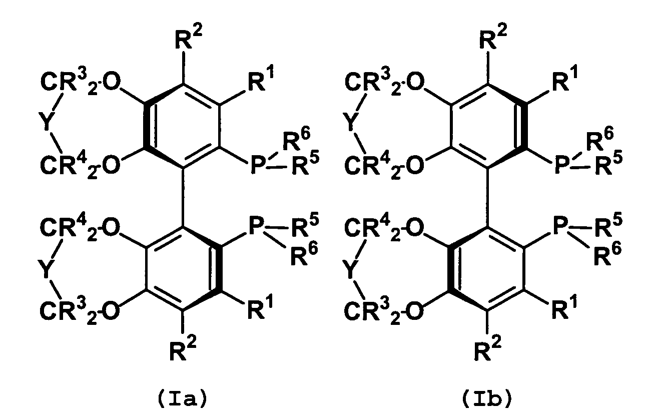

3. Process for preparing compounds according to claim 1, which comprises the following steps:
A) reaction of a compound of the general formula (IV) 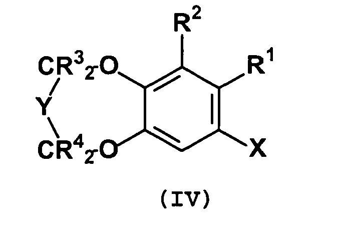 where X is halogen and
R¹, R², R³, R⁴, R⁷, R⁸, R⁹, R¹⁰, Q and Y are as defined in claim 1,
with a phosphinyl chloride R⁵R⁶P(O)Cl, where R⁵ and R⁶ are as defined in claim 1, to form a compound of the general formula (IIIa) 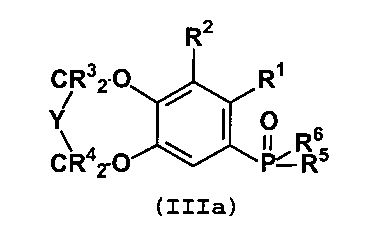
B) conversion of the compound of the general formula (IIIa) obtained in step A into a compound of the general formula (II) 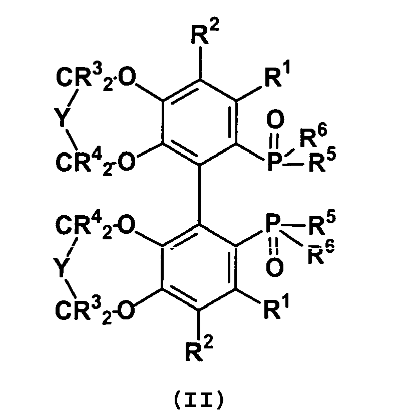 by oxidative coupling and
C) reduction of the compound of the general formula (II) obtained in step B.

4. Process according to claim 3, **characterized in that**, in step B, the compound of the general formula (IIIa) obtained from step A is firstly converted by iodation into an intermediate of the general formula (IIIb) 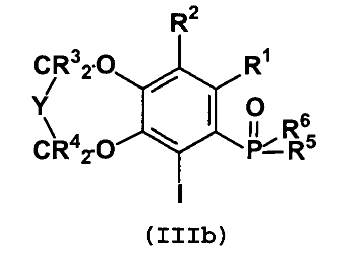 and this is subsequently converted in a metal-catalyzed coupling reaction into a compound of the general formula (II) according to claim 2.

5. Process according to claim 3 or 4, **characterized in that** the compounds of the general formula (II) obtained in step B are prepared in enantiomerically pure or enantiomerically enriched form corresponding to the general formulae (IIa) and (IIb) 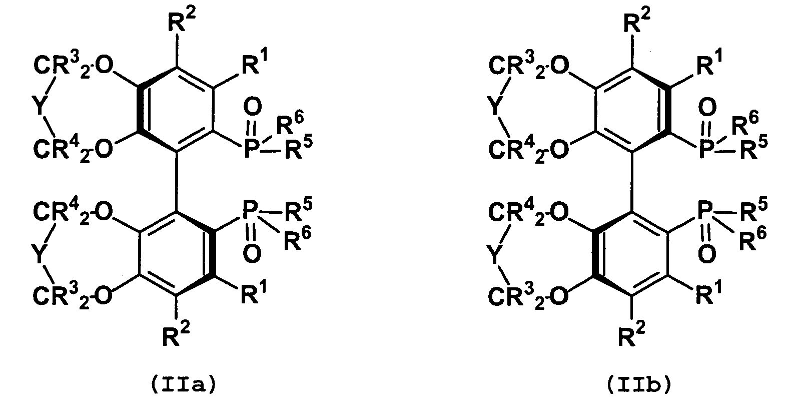 by fractional crystallization in the presence of complexing chiral compounds.

6. Process according to claim 3 or 4, **characterized in that** the compounds of the general formula (I) obtained are prepared in enantiomerically pure or enantiomerically enriched form corresponding to the general formulae (Ia) and (Ib) by means of conversion into chiral palladium complexes in an additional step D.

7. Compounds of the general formula (II) 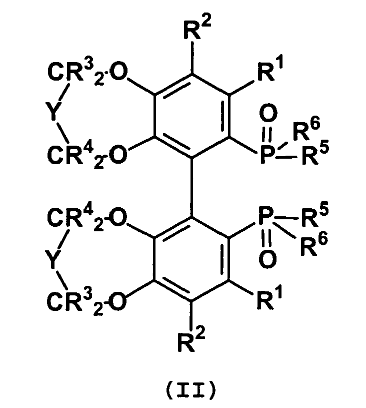 **characterized in that**
R¹, R², R³, R⁴, R⁵, R⁶, R⁷, R⁸, R⁹, R¹⁰, Q and Y are as defined in claim 1.

8. Compounds according to claim 7 in the optical configuration of the general formulae (IIa) and (IIb)

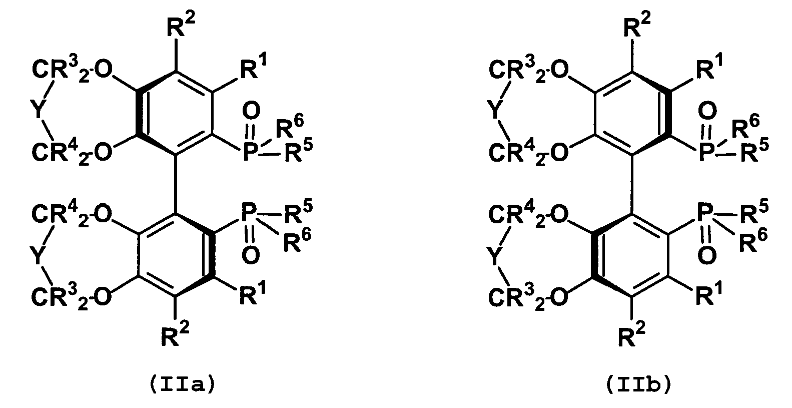

9. Process for preparing enantiomerically pure or enantiomerically enriched compounds according to claim 8 by fractional crystallization of compounds according to claim 7 in the presence of complexing chiral compounds.

10. Process for preparing enantiomerically pure or enantiomerically enriched compounds according to claim 2 by means of conversion of compounds according to claim 1 into chiral palladium complexes.

11. Compounds of the general formula (IIIa) or (IIIb) 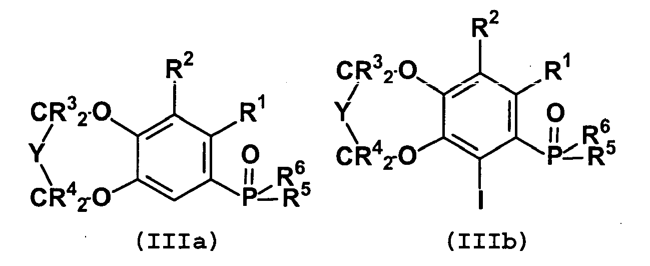 **characterized in that**
R¹, R², R³, R⁴, R⁵, R⁶, R⁷, R⁸, R⁹, R¹⁰, Q and Y are as defined in claim 1.

12. Use of compounds of the general formula (I) as ligands for preparing complexes comprising at least one ligand of the general formula (I) and at least one metallic center.

13. Complexes comprising at least one ligand of the general formula (I) and at least one metallic center.

14. Complexes according to claim 13, **characterized in that** at least one ligand of the general formula (I) is present in enantiomerically pure or enantiomerically enriched form.

15. Complexes according to claim 13 or 14, **characterized in that**, in the case of a ligand of the general formula (I) which is not enantiomerically pure being present, another chiral ligand is additionally present.

16. Complexes according to one or more of claims 13 to 15, **characterized in that** the metallic center is selected from the group consisting of rhodium, ruthenium, iridium, palladium, copper and nickel.

17. Process for preparing complexes according to claim 13 by reacting a compound of the general formula (I) with a precursor containing the metallic center in the presence of an organic solvent.

18. Use of the complexes according to one or more of claims 13 to 16 as catalysts in organic synthesis.

19. Use according to claim 18, **characterized in that** a chiral complex is used as catalyst in asymmetric organic synthesis.

20. Use according to claim 18 or 19 as homogeneous catalyst or in immobilized form as heterogeneous catalyst in organic synthesis.

21. Use according to one or more of claims 18 to 20 as catalyst in hydrogenation, isomerization and C-C bond formation reactions.

22. Process for hydrogenating C=O, C=C or C=N groups in a substrate, **characterized in that** the hydrogenation is carried out in the presence of a complex according to any of claims 13 to 17.

23. Process according to claim 22, **characterized in that** an asymmetric hydrogenation is carried out in the presence of a chiral complex according to claim 15 or 16.

24. Process according to claim 23, **characterized in that** the hydrogenation is carried out in the presence of a complex containing (*S*)-(-)-[6,6'-bis(3,4-dihydro-2*H*-1,5-benzodioxepin)-7,7'-diyl]bis(diphenylphosphine) (**VIIa**) 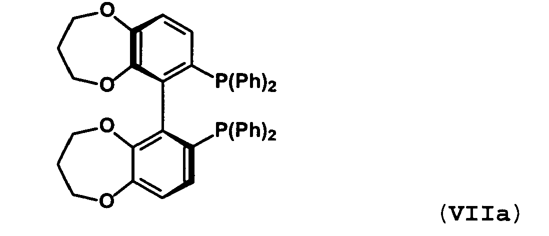 or (R)-(+)-[6,6'-bis(3,4-dihydro-2*H*-1,5-benzodioxepin)-7,7'-diyl]bis(diphenylphosphine) **(VIIb)** 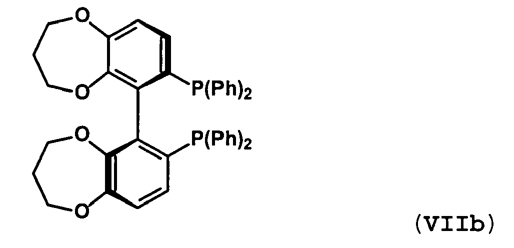 as ligand.

## Revendications

1. Composés de formule générale (I) 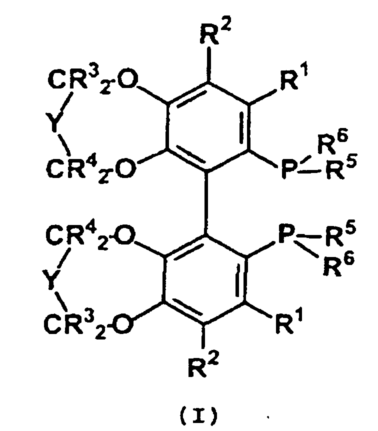 **caractérisés en ce que**
**R**^{**1**} et **R**^{**2**} représentent un atome d'hydrogène et
les radicaux **R**^{**3**} sont identiques et sont choisis dans l'ensemble constitué par les atomes d'hydrogène et de fluor, les groupes alkyle en C₁-C₁₀ et CF₃,
les radicaux **R**^{**4**} sont identiques et sont choisis dans l'ensemble constitué par les atomes d'hydrogène et de fluor, les groupes alkyle en C₁-C₁₀ et CF₃,
**Y** représente un radical divalent chois dans l'ensemble constitué par les groupes CR⁹₂, CHR⁹, (*cis*) -CH=CH, CR⁹₂CR¹⁰₂, CHR⁹CHR¹⁰, 1,2-arylène, CHR⁹ -O-CHR¹⁰ et CR⁹₂-O-CR¹⁰₂,
**R**^{**9**} et **R**^{**10**} étant identiques ou différents et étant par ailleurs choisis, indépendamment l'un de l'autre, dans l'ensemble constitué par l'atome d'hydrogène, **Q,** des groupes alkyle en C₁-C₁₀, cycloalkyle en C₃-C₁₀, alcényle en C₂-C₁₀, cycloalcényle en C₄-C₁₀, alcynyle en C₂-C₁₀, aryle en C₆-C₁₅ et hétéroaryle en C₁-C₁₅ non substitués ou une ou plusieurs fois substitués, les substituants pouvant pour leur part avoir éventuellement la signification de **Q** et
**Q** est choisi dans le groupe contenant -F, -Cl, -Br, -I, -CN, -NO₂, -NR⁷R⁸, NR⁷OR⁸, -OR⁷, -C(O)R⁷, SR⁷, -SO₃R⁷, -C(O)OR⁷, -C(O)NR⁷R⁸, -OC(O)R⁷, -NR⁷C(O)R⁸,
**R**^{**7**} et **R**^{**8**} étant identiques ou différents et pouvant par ailleurs avoir, indépendamment l'un de l'autre, la signification de **R**^{**9**}**,**
**R**^{**5**} et **R**^{**6**} pouvant être identiques ou différents et étant choisis, indépendamment l'un de l'autre, dans l'ensemble constitué par des groupes cycloalkyle en C₃-C₁₀, cycloalcényle en C₄-C₁₀, aryle en C₅-C₁₅ ou hétéroaryle en C₁-C₁₅ non substitués ou une ou plusieurs fois substitués, les substituants pouvant pour leur part avoir éventuellement la signification de **Q**.

2. Composés selon la revendication 1, en la configuration optique des formules générales (Ia) et (Ib)

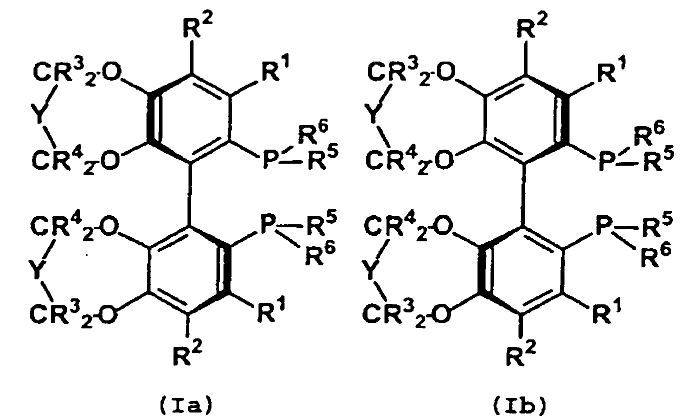

3. Procédé pour la préparation des composés selon la revendication 1, comprenant les étapes suivantes :
(A) mise en réaction d'un composé de formule générale (IV) 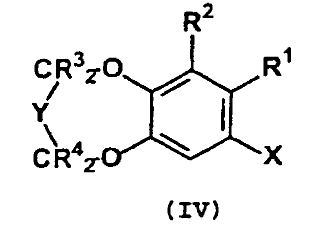 dans laquelle X représente un atome d'halogène et
**R**^{**1**}**, R**^{**2**}**, R**^{**3**}**, R**^{**4**}**, R**^{**7**}**, R**^{**8**}**, R**^{**9**}**, R**^{**10**}**, Q** et **Y** ont les significations données dans la revendication 1,
avec un chlorure de phosphinyle R⁵R⁶P(O)Cl, **R**^{**5**} et **R**^{**6**} ayant les significations données dans la revendication 1, pour l'obtention d'un composé de formule générale (IIIa) 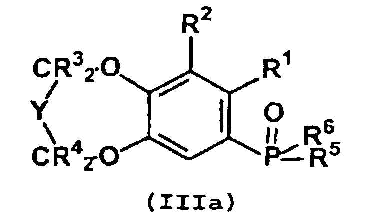
B) conversion du composé de formule générale (IIIa), obtenu selon l'étape A, en un composé de formule générale (II) 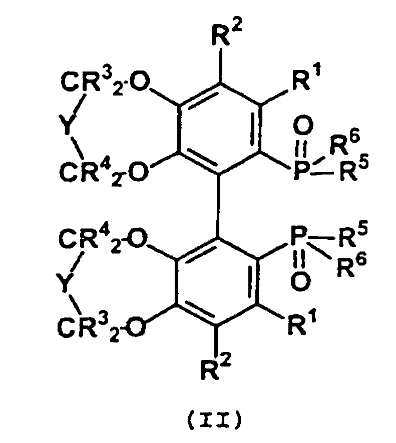 par couplage oxydant et
C) réduction du composé de formule générale (II), obtenu selon l'étape B.

4. Procédé selon la revendication 3, **caractérisé en ce que** dans l'étape B d'abord on convertit par iodation le composé de formule générale (IIIa), obtenu dans l'étape A, en un produit intermédiaire de formule générale (IIIb) 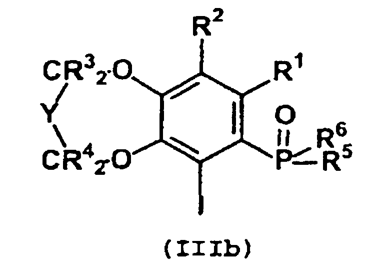 et ensuite on convertit ce dernier, dans une réaction de couplage catalysée par un métal, en un composé de formule générale (II) selon la revendication 2.

5. Procédé selon la revendication 3 ou 4, **caractérisé en ce que** les composés de formule générale (II), obtenus selon l'étape B, sont préparés par cristallisation fractionnée en présence de composés chiraux complexants sous forme d'énantiomères pur ou enrichie en énantiomères, de formules générales (IIa) et (IIb)

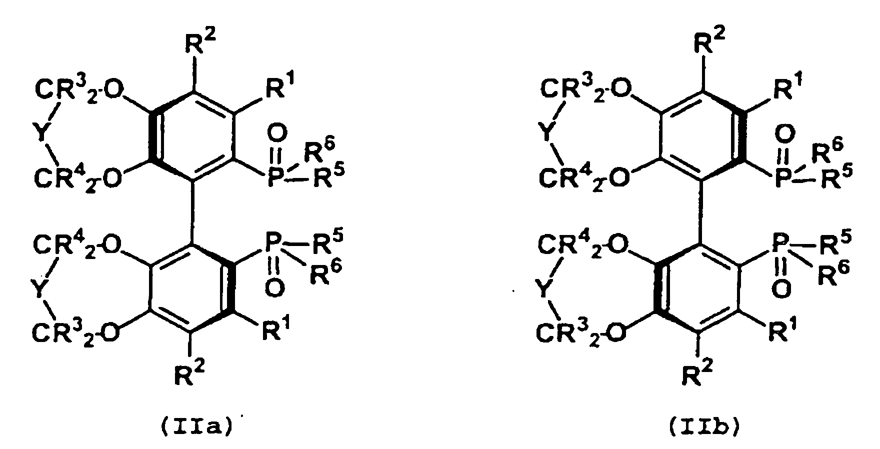

6. Procédé selon la revendication 3 ou 4, **caractérisé en ce que** dans une étape D supplémentaire les composés de formule générale (I) obtenus sont préparés par conversion en complexes chiraux de palladium sous forme d'énantiomères purs ou enrichie en énantiomères, de formules générales (Ia) et (Ib).

7. Composés de formule générale (II) 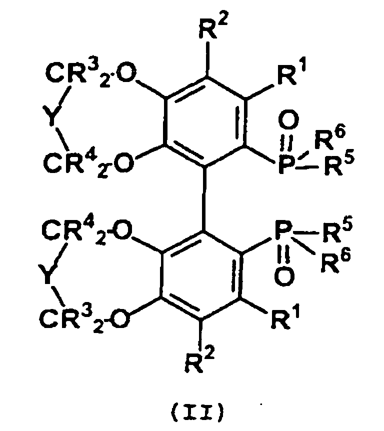 **caractérisés en ce que**
**R**^{**1**}**, R**^{**2**}**, R**^{**3**}**, R**^{**4**}**, R**^{**5**}**, R**^{**6**}**, R**^{**7**}**, R**^{**8**}**, R**^{**9**}**, R**^{**10**}**, Q** et **Y** ont les significations données dans la revendication 1,

8. Composés selon la revendication 7, en la configuration optique des formules générales (Ia) et (Ib)

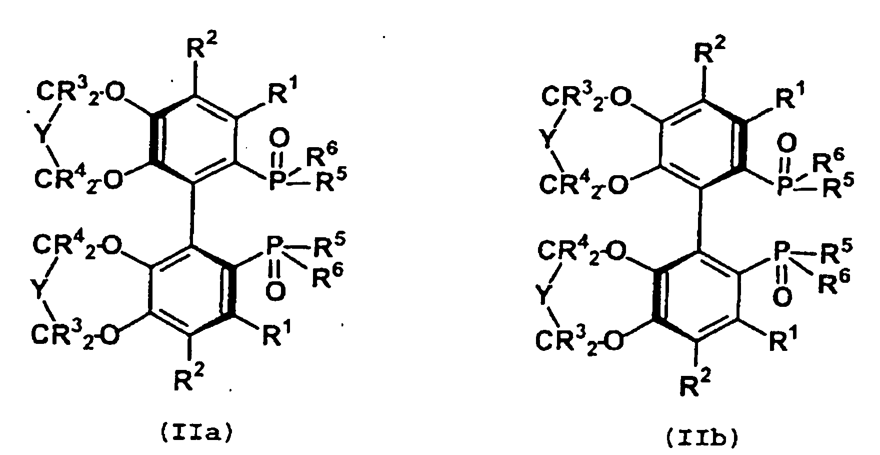

9. Procédé pour la préparation de composés sous forme d'énantiomères purs ou enrichis en énantiomères, selon la revendication 8, par cristallisation fractionnée de composés selon la revendication 7 en présence de composés chiraux complexants.

10. Procédé pour la préparation de composés sous forme d'énantiomères purs ou enrichis en énantiomères, selon la revendication 2, par conversion de composés selon la revendication 1 en complexes chiraux de palladium.

11. Composés de formule générale (IIIa) ou (IIIb) 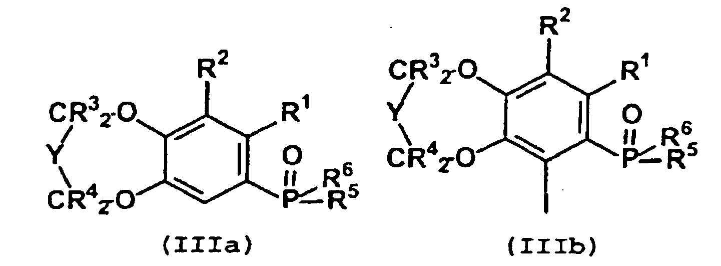 **caractérisés en ce que**
**R**^{**1**}**, R**^{**2**}**, R**^{**3**}**, R**^{**4**}**, R**^{**5**}**, R**^{**6**}**, R**^{**7**}**, R**^{**8**}**, R**^{**9**}**, R**^{**10**}**, Q** et **Y** ont les significations données dans la revendication 1.

12. Utilisation des composés de formule générale (I) en tant que ligands pour la préparation de complexes contenant au moins un ligand de formule générale (I) et au moins un centre métallique.

13. Complexes contenant au moins un ligand de formule générale (I) et au moins un centre métallique.

14. Complexes selon la revendication 13, **caractérisés en ce qu'**au moins un ligand de formule générale (I) est contenu sous forme d'énantiomère pur ou sous forme enrichie en énantiomère.

15. Complexes selon la revendication 13 ou 14, **caractérisés en ce que** dans le cas où un ligand de formule générale (I) non sous forme d'énantiomère pur est contenu, on ajoute en plus un autre ligand chiral.

16. Complexes selon une ou plusieurs des revendications 13 à 15, **caractérisés en ce que** le centre métallique est choisi dans le groupe comprenant le rhodium, le ruthénium, l'iridium, le palladium, le cuivre et le nickel.

17. Procédé pour la préparation de complexes selon la revendication 13, par mise en réaction d'un composé de formule générale (I) avec un précurseur contenant le centre métallique, en présence d'un solvant organique.

18. Utilisation des complexes selon une ou plusieurs des revendications 13 à 16, en tant que catalyseurs en synthèse organique.

19. Utilisation selon la revendication 18, **caractérisée en ce qu'**un complexe chiral est utilisé comme catalyseur dans la synthèse organique asymétrique.

20. Utilisation selon la revendication 18 ou 19, en tant que catalyseur homogène ou, sous forme immobilisée, en tant que catalyseur hétérogène, en synthèse organique.

21. Utilisation selon une ou plusieurs des revendications 18 à 20, en tant que catalyseur dans des réactions d'hydrogénation, d'isomérisation et de jonction C-C.

22. Procédé pour l'hydrogénation de groupes C=O, C=C ou C=N dans un substrat, **caractérisé en ce qu'**on effectue l'hydrogénation en présence d'un complexe selon les revendications 13 à 17.

23. Procédé selon la revendication 22, **caractérisé en ce qu'**on effectue une hydrogénation asymétrique en présence d'un complexe chiral selon la revendication 15 ou 16.

24. Procédé selon la revendication 23, **caractérisé en ce qu'**on effectue l'hydrogénation en présence d'un complexe contenant de la (S) - (-) - [6, 6' -bis- (3, 4-dihydro-2H-1,5-benzodioxépine)-7,7'-diyl]bis-(diphénylphosphine) (**VIIa**) 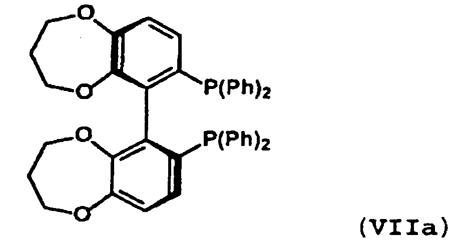 ou de la (R)-(+)-[6,6'-bis-(3,4-dihydro-2H-1,5-benzodioxépine)-7,7'-diyl]bis(diphénylphosphine) (**VIIb**) 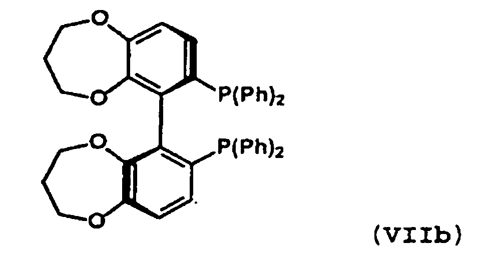 en tant que ligand.
